# EUROPEAN PATENT APPLICATION

(11) **EP 4 491 192 A1**
(43) Date of publication of application: **15.01.2025**
(21) Application number: 23382727.8
(22) Date of filing: 14.07.2023
(51) Int. Cl.: A61K 39/00, C07K 16/28

(54) **ANTIBODY AGAINST PRE-T CELL RECEPTOR ALPHA, ANTIBODY-DRUG CONJUGATES AND CAR-T CELLS, FOR THE TREATMENT AND/OR PREVENTION OF LEUKEMIA**

(71) Applicant: Consejo Superior De Investigaciones Científicas, 28006 Madrid (ES)
(72) Inventor: Toribio García, Mª Luisa, Madrid (ES); García Peydró, Marina, Madrid (ES); Fuentes Villarejo, Patricia, Madrid (ES); González García, Sara, Madrid (ES); Bayón Calderón, Fátima, Madrid (ES); Cela Rodríguez, Carmela, Madrid (ES); Alcain Sánchez, Juan, Madrid (ES); Castillo Gutiérrez, Eloísa, Madrid (ES)
(74) Representative: Pons

(57) **Abstract**

The present invention relates to an antibody, or an antigen binding fragment thereof, and to the CAR T-cell comprising the antigen binding fragment of said antibody. The invention also discloses to the polynucleotides encoding said antibody, the vector, the cells, the antibody-drug conjugate, and the composition comprising the antibody, the antigen binding fragment thereof, and/or to the CAR T-cell, all of them being useful in the treatment and/or prevention of leukemia, more preferably T-cell acute lymphoblastic leukemia (T-ALL), still more preferably T-ALL pre-TCR+, in a subject.

## Description

The present invention provides compounds and compositions for use in the treatment/prevention of cancer, specifically human acute lymphoblastic leukemia (ALL) of the T-cells (T-ALL), as part of an antibody-Drug Conjugate or a Chimeric Antigen Receptor T-cell (CAR-T) therapy. Thus, the present invention relates to belongs to the chemistry and pharmacy sector including the treatment and/or prevention of said disease.

### BACKGROUND ART

The most common cause of childhood morbidity is acute lymphoblastic leukemia (ALL), an aggressive tumor arising from oncogenic transformation of T or B lymphoid progenitors during their development (T-ALL and B-ALL leukemia, respectively), of which T-ALL constitutes 10-15% of the cases in children and 20-25% in adults (Karrman and Johansson, Genes Chromosomes Cancer, 56 (2), 89-116, February 2017).

ALL, particularly T-ALL, has a poor prognosis, although the development of intensive chemotherapy treatments in recent decades has notably increased the life expectancy of patients with ALL of both cell types. The 5-year survival rate for T-ALL patients is over 70%. In cases of relapses, the survival rate of T-ALL patients drops drastically to as low as 20%.

Most ALL relapses occur in early post-treatment phases and reflect the treatment resistance of a clonal population of cells that were already present at diagnosis (Bailey et al., Lancet Oncol. 5 9: 873-883. 2008), and are responsible for the onset, spread, and persistence of leukemia, hence the name "leukemia-initiating cells" (LICs). The success of ALL treatment will therefore depend on the efficient elimination of LICs. Although significant efforts have been made to design new treatments that fulfill this objective, the development of specific anti-LIC drugs which reduce the frequency of relapses is still in its initial phases. This work is complex, since LICs represent less than 1% of the total leukemic population and specific markers for these cells have not yet been found. Accordingly, the success of any new therapy depends on the development of strategies which allow the LICs of T-ALL and B-ALL to be identified and characterized, so as to promote the design of new tactics allowing their elimination.

T-lymphocytes interact with antigens through the T-cell antigen receptor (TCR, for T-Cell Receptor) which recognizes its target antigen processed and presented in the form of peptides by major histocompatibility complex (MHC) molecules. This TCR receptor is a specific heterodimer of each T-cell clone which is formed by a TCRα polypeptide chain and a TCRβ polypeptide chain expressed on the surface associated with the CD3 complex (TCRαβ). About 90-95% of peripheral blood T-cells are Taβ cells that express a TCRaβ. The remaining 5-10% (Tγδ cells) express a TCR formed by a TCRγ chain and a TCRδ chain. Each of these TCR chains is different in each T-cell clone and is made up of a unique combination of domains referred to as variable (V), [diversity (D)], binding (J), and constant (C) domains. In each T-cell clone, the combination of the V, (D), and J domains in both α and β chains or in both γ and δ chains participates in antigen recognition in a specific manner through 3 complementary regions (CDR for Complementarity Determining Region), mainly through CDR3.

Like immunoglobulin genes, TCR receptor genes consist of a series of regions encoding the V, D, J, and C domains that are rearranged during T-cell development in the thymus. Intrathymic progenitors of Taβ lymphocytes have to go through at least two selection processes mediated by signals through two different receptors, the pre-T cell receptor or pre-TCR, and the mature receptor or TCRαβ, that are expressed sequentially during intrathymic development (Borst et al., Curr Opin Immunol 1996; 8: 181-90). In the thymus, genes encoding the TCRβ chain are rearranged and expressed before those corresponding to the TCRα locus. Thymocytes that productively rearrange the TCRβ locus first express a pre-TCR complex, independent of TCRα, made up of a TCRβ chain covalently linked to an invariant pre-TCRa chain (pTa), which associates with the CD3 complex and promotes a quality control process generally known as "β-selection"(Groettrup et al., Cell 1993; 75: 283-94). β selection induces the survival and proliferative expansion of those thymocytes in which correct TCRβ rearrangement takes place and simultaneously determines the inhibition of other rearrangements in this locus or allelic exclusion (Dudley et al., Immunity 1994; 1: 83-93). Cell expansion induced by pre-TCR ends abruptly after its internalization and degradation, leading to a population of resting thymocytes where rearrangements at the TCRα locus are then initiated (Petry et al., Exp Med 1993; 178: 615-22). Following the expression of TCRα and the substitution of pTa with TCRα, the TCRαβ receptor is expressed in association with CD3 on the cell membrane, and thymocytes undergo a second selection process known as "positive selection", are rescued from programmed cell death, and the final differentiation of these pre-T cells into mature Taβ cells takes place. By means of an additional "negative selection" process, clonal elimination of those thymocytes with self-reactive TCRαβ receptors will be induced, thereby ensuring tolerance of the individual to the components themselves.

A major advancement in the treatment of ALL has been the incorporation of targeted therapy by means of mAb, such as anti-CD20, anti-CD19, and anti-CD22 mAbs, among others. These mAbs are directed against malignant B-cells, but they also recognize normal B-cells that are eliminated along with tumor cells, therefore, it is necessary to infuse patients with immunoglobulins. In the case of T-ALL, there are some studies conducted with anti-CD52 mAb which recognizes both T- and B-cells, with not very encouraging results and considerable side effects due to the elimination of normal T-cells, which leads to T-cell aplasia and to the onset of a severe life-threatening immunodeficiency (Angiolillo et al., Pediatr Blood Cancer. 2009; 53:978-83).

In that sense, there is a need in the state of the art for alternative and more effective management solutions for the treatment of leukemia, particularly of T-ALL. Adoptive T cell transfer (ACT) is a new area of transfusion medicine involving the infusion of lymphocytes to mediate antitumor, antiviral, or anti-inflammatory effects. The field has rapidly advanced from a promising form of immuno-oncology in preclinical models to the recent commercial approvals of chimeric antigen receptor (CAR) T cells to treat leukemia and lymphoma. CAR-T cells are T-cells genetically modified to express on the surface a receptor which recognizes the tumor, and become activated to eliminate same. A CAR combines antigen-binding domains, most commonly, a single-chain variable fragment (scFv) derived from the variable domains of antibodies with the signaling domains of the CD3-ζ chain and additional costimulatory domains from receptors such as CD28, OX40, and CD137.

An example of such therapies is disclosed in US2020010555A1 which describes an anti-CD123 CAR-T immunotherapy treatment, with B-ALL being one of the treatment targets. Another example of immunotherapy targeting B-ALL and other B-cell leukemias is disclosed in document US2019062430 which refers specifically to CD19 CAR-T immunotherapy. These treatments improve the survival rate by 50 - 76% in cases of B-ALL relapses, but their efficacy in treating T-ALL has yet to be proven and appears doubtful at the moment because no specific targets of tumor T-cells, which prevent the elimination of normal T-cells and the fratricide of CAR-T, have been identified (Tandon and Punnett, Advances in Hematologic Malignancies, Gamal Abdul Hamid, IntechOpen, 23 October 2019).

Therefore, the development of alternative therapeutic agents for the treatment of ALL, particularly T-ALL, which are directed against new therapeutic targets selectively involved in the disease, is crucial.

### DESCRIPTION OF THE INVENTION

The inventors have observed that a single chain Fv (scFv) antibody comprising the complementarity-determining regions (CDRs) of sequence SEQ ID NO: 1, SEQ ID NO: 2, SEQ ID NO: 3, SEQ ID NO: 4, SEQ ID NO: 5 and SEQ ID NO: 6, is capable of specifically binding the human pre-TCR receptor. In addition, if this scFv is incorporated into a CAR expressed in a T cell, the specific recognition of the pre-TCR complex by the CAR-T cell triggers a robust *in vitro* cytotoxic activity, resulting in an elimination of pre-TCR+ leukemic cells (SupT1). Therefore, the present invention provides an agent useful in the treatment and/or prevention of human T-ALL, particularly of pre-TCR+ T-ALL, including the treatment and/or prevention of relapses of said disease.

Thus, in one aspect, the present invention relates to an isolated antibody, or an antigen binding fragment thereof, comprising a heavy chain (HC) immunoglobulin variable domain sequence and a light chain (LC) immunoglobulin variable domain sequence, hereinafter "antibody of the invention") wherein
the HC comprises
   (a) a CDR1 comprising, or consisting of, the amino acid sequence RYAMS (SEQ ID NO: 1) or a functionally equivalent variant thereof; and
   (b) a CDR2 comprising, or consisting of, the amino acid sequence AAISSGGSYTYYPDSVKG (SEQ ID NO: 2) or a functionally equivalent variant thereof; and
   (c) a CDR3 comprising, or consisting of, the amino acid sequence of LRVVAEGGSYFDY (SEQ ID NO: 3) or a functionally equivalent variant thereof; and
the LC comprises:
   (a) a CDR1 comprising, or consisting of, the amino acid sequence of RSSQSILHSNGNTYLE (SEQ ID NO: 4) or a functionally equivalent variant thereof; and
   (b) a CDR2 comprising, or consisting of, the amino acid sequence of KVSNRFS (SEQ ID NO: 5) or a functionally equivalent variant thereof; and
   (c) a CDR3 comprising, or consisting of, the amino acid sequence of FQGSHVPVT (SEQ ID NO: 6) or a functionally equivalent variant thereof.

In a particular embodiment of the antibody of the invention, the HC immunoglobulin variable domain comprises, or consists of, the amino acid sequence SEQ ID NO: 7, or a functionally equivalent variant thereof, and/or LC immunoglobulin variable domain comprises, or consists of, the sequence SEQ ID NO: 8, or a functionally equivalent variant thereof.
SEQ ID NO: 7 
EVQLVESGGGLVKPGGSLQLSCAASGFTFNRYAMSWVRQTPEKRLEWVAAISSG
GSYTYYPDSVKGRFIISRDNAKNTLSLQMSSLRSEDTAMFYCARLRVVAEGGSYFD
YWGQGTTLTVSS
SEQ ID NO: 8

DVLMTQTPLSLPVSLGDQASISCRSSQSILHSNGNTYLEWYLQKPGQSPKLLIYKV
SNRFSGVPDRFSGSGSGTDFTLKISRVEAEDLGVYYCFQGSHVPVTFGSGTKLEIK

Underlined: antibody CDRs.

Persons skilled in the art understand that mutations in the nucleotide sequence of genes that lead to conservative amino acid substitutions at non-critical positions for the functionality of the protein are evolutionarily neutral mutations which do not affect its global structure or its functionality, giving rise to proteins which although comprise different amino acid sequence perform the same activity. These proteins are considered "functionally equivalent variants" of the sequence corresponding sequence (in the present invention, the sequences SEQ ID NO: 1 to SEQ ID NO: 8) and fall within the scope of the present invention. Thus, the term "functionally equivalent variant", as used herein, means an amino acid sequence which is derived from a native sequence (SEQ ID NO: 1 to SEQ ID NO: 8 in the present invention) by one or more deletions, insertions and/or substitutions of one or more amino acids at site(s) within its amino acid sequence and performs the same activity, i.e. can act as antigen binding domain and specifically recognize and bind to a target antigen. Functionally equivalent variants can be prepared using any mutagenesis procedure known in the art, such as site-directed mutagenesis, synthetic gene construction, semi-synthetic gene construction, random mutagenesis, shuffling, etc. An assay to test if a given amino acid sequence is a functionally equivalent fragment of the sequences SEQ ID NO: 1 to SEQ ID NO: 8 is disclosed in the examples accompanying the present description.

As used herein, the terms "antibody", "antibodies", "ab", "Ab" or "immunoglobulin" are used interchangeably in the broadest sense as used in the present invention. The term "antibody" refers to immunoglobulin molecules and immunologically active portions (or fragments) of immunoglobulin molecules (also called "antigen binding fragments"). That is, it refers to molecules that specifically bind (are immunoreactive) to an antigen, such as, for example, a peptide or a protein (also called an immunogen or epitope). The term "epitope" is a region of an antigen that is bound by an antigen binding protein, including antibodies. In the present invention, the antigen is the pre-T cell receptor alpha (pT alpha or pTα), preferably human pTα, of the receptor expressed during the development of T-lymphocytes, known as pre-TCR (pre-T cell receptor).

In terms of antibody structure, an immunoglobulin has heavy (H) chains and light (L) chains interconnected by disulfide bonds. There are two types of light chain, lambda (λ) and kappa (κ). There are five main heavy chain classes (or isotypes) which determine the functional activity of an antibody molecule: IgM, IgD, IgG, IgA and IgE. Each heavy and light chain contains a constant region and a variable region, (the regions are also known as "domains"). In combination, the heavy and the light chain variable regions specifically bind the antigen. Light and heavy chain variable regions contain a "framework" region interrupted by three hypervariable regions, also called "complementarity-determining regions" or "CDRs".

The CDRs are primarily responsible for binding to an epitope of an antigen. The CDRs of each chain are typically referred to as CDR1, CDR2, and CDR3, numbered sequentially starting from the N-terminus, and are also typically identified by the chain in which the particular CDR is located. Thus, a VH CDR3 is located in the variable domain of the heavy chain of the antibody in which it is found, whereas a VL CDR1 is the CDR1 from the variable domain of the light chain of the antibody in which it is found. An antibody that binds pTa will have a specific VH region and the VL region sequence, and thus specific CDR sequences.

In the context of the present invention, the term antibody encompasses both monoclonal and polyclonal antibodies. Preferably, in the present invention the antibody is monoclonal. As use herein, "Monoclonal antibodies" (or mAbs) are homogenous populations of identical antibodies, produced by a hybridoma, that is, a hybrid cell that is the product of the fusion of a clone of B lymphocytes descendant of a single and unique stem cell and a plasma cell tumor, which are directed against a specific site or antigenic determinant.

As explained above, the present invention also encompasses immunologically active portions (or fragments) of immunoglobulin molecules, also called herein "antigen binding fragments". Examples of "antigen binding fragments" are selected from the group consisting of Fv, scFv (sc for single chain), Fab, F(ab')2, Fab', scFv-Fc fragments or diabodies, or any fragment of which the half-life time would have been increased by chemical modification, such as the addition of poly(alkylene) glycol such as poly(ethylene) glycol ("PEGylation") (pegylated fragments called Fv-PEG, scFv-PEG, Fab-PEG, F(ab')2-PEG or Fab'-PEG) ("PEG" for Poly(Ethylene) Glycol), or by incorporation into a liposome, wherein said fragments specifically comprises the six above-mentioned CDRs (SEQ ID NO: 1 to SEQ ID NO: 6). The "antigen binding fragments" will comprise a partial sequence of the heavy or light variable chain of the antibody from which they are derived, said partial sequence being sufficient to retain the same specificity of binding as the antibody from which it is descended and a sufficient affinity, preferably at least equal to 1/100, in a more preferred manner to at least 1/10, of the affinity of the antibody from which it is descended, with respect to the target. In another particular embodiment of the antibody of the invention, the antibody is selected from the group consisting of a monoclonal antibody, a chimeric antibody, a humanized antibody, or the "antigen binding domain" is selected from a Fab, a F(ab')2, a Fab', a scFv, and a Fv.

In a more preferred embodiment, the antibody of the invention is conjugated to a detectable marker.

Said "conjugation" can be carried out by means of any method known in the art to functionally connect the domains of an antibody with a marker producing a signal which can be visually detected including, without limitation, the recombinant fusion with or without intermediate domains, antibody-mediated fusion, non-covalent association and covalent bonding, for example, the binding of the antibody to the disulfide, hydrogen bonding, electrostatic bonding, and conformational bonding, for example, biotin-avidin associations. Conjugation with a label can be by chemical or recombinant means. Chemical means refers to a reaction between the antibody and the label or marker such that a covalent bond is formed between the two molecules in order to form a single molecule.

The term "detectable marker or label", as it is used herein, refers to molecules that can generate a signal which is detectable by means of a method suitable for said detection. Marker detection methods include, among others, fluorescence, light, confocal and electron microscopy, magnetic resonance and spectroscopy; fluoroscopy, computerized tomography and positron emission tomography, enzyme-linked immunosorbent assays, size exclusion chromatography by fluorescence detection, lateral flow test, radioimmunoassay, radiolabeling, Western blot, immunohistochemistry, immunofluorescence, immunocytochemistry, flow cytometry, fluorescence-activated cell sorting, immunoprecipitation, and enzyme-type immunospot. Suitable markers include, among others, fluorescein, rhodamine, eosin and other fluorophores, radioisotopes, gold, gadolinium and other lanthanides, paramagnetic iron, fluorine-18 and other positron-emitting radionuclides. Furthermore, the probes or labels may be bi- or multifunctional and can be detected by means of more than one of the listed methods. The marker or label can be, for example, a chromogenic, fluorogenic, magnetic, electrodense, radioactive, and/or chemiluminescent compound. In a preferred embodiment of the present invention, the detectable label or marker is selected from the list consisting of: fluorescein isothiocyanate (FITC), allophycocyanin (APC), or any other relevant fluorescent dye in flow cytometry, biotin, horseradish peroxidase (HRP), gold beads, and paramagnetic iron.

On the other hand, therapies based on the use of monoclonal antibodies, or antigen binding fragments thereof, attached to toxins (giving rise to Antibodies Drug Conjugates (ADC)) are particularly efficient in cases of internalization of the corresponding molecular targets. In that sense, in another preferred embodiment, the antibody, or an antigen binding fragment thereof, of the invention is coupled to a toxin.

The term "coupled", as it is used in the present invention, refers to the fact that the antibody is functionally connected or conjugated to the toxin, including without limitation, the recombinant fusion with or without intermediate domains, antibody-mediated fusion, non-covalent association and covalent bonding, for example, the binding of the antibody to the disulfide, hydrogen bonding, electrostatic bonding, and conformational bonding, for example, biotin-avidin associations. Coupling with a toxin can be by chemical or recombinant means. Chemical means refers to a reaction between the antibody and the toxin such that a covalent bond is formed between the two molecules in order to form a single molecule. Alternatively, the antibody and the toxin can be conjugated by means of a linker or connector. The toxin coupled to the antibody can be selected, without limitations, from the list consisting of: cytotoxins or cytostatic agents, radioactive or radiolabeled molecules, chemotherapeutic drugs and agents, or any combination thereof. Particular examples of toxin that may be used in the present invention are Mertansine Toxin (DM1) o Monomethyl auristatin E toxin (MMAE).

As the skilled person in the art understands, antigen binding fragments may be used to develop CAR T-cells. CAR T-cells are genetically engineered T-cells in which single chain antibody fragments (scFv) or ligands are attached to the T-cell signaling domains capable of facilitating T-cell activation. CARs combine HLA-independent targeting specificity of a monoclonal antibody with the cytolytic activity and homing properties of activated T-cells.

Therefore, in another aspect, the present invention relates to a chimeric antigen receptor (CAR), hereinafter "CAR of the invention", comprising:
(a) an extracellular domain comprising, or consisting of, a heavy chain (HC) immunoglobulin variable domain sequence and a light chain (LC) immunoglobulin variable domain sequence, wherein
   the HC comprises
      (i) a CDR1 comprising, or consisting of, the sequence SEQ ID NO: 1 or a functionally equivalent variant thereof; and
      (ii) a CDR2 comprising, or consisting of, the sequence SEQ ID NO: 2 or a functionally equivalent variant thereof; and
      (iii) a CDR3 comprising, or consisting of, the sequence SEQ ID NO: 3 or a functionally equivalent variant thereof; and
   the LC comprises:
      (i) a CDR1 comprising, or consisting of, the sequence SEQ ID NO: 4 or a functionally equivalent variant thereof; and
      (ii) a CDR2 comprising, or consisting of, the sequence SEQ ID NO: 5 or a functionally equivalent variant thereof; and
      (iii) a CDR3 comprising, or consisting of, the sequence SEQ ID NO: 6 or a functionally equivalent variant thereof;
(b) a transmembrane domain; and
(c) at least one endodomain.

In a particular embodiment of the CAR of the invention, the HC immunoglobulin variable domain sequence comprises, or consists of, the sequence SEQ ID NO: 7, or a functionally equivalent variant thereof, and the LC immunoglobulin variable domain sequence comprises, or consists of, the sequence SEQ ID NO: 8, or a functionally equivalent variant thereof.

The term "chimeric antigen receptor" (CAR), as used herein, refers to a fused protein comprising an extracellular domain capable of binding to an antigen, a transmembrane domain derived from a polypeptide different from a polypeptide from which the extracellular domain is derived, and at least one intracellular domain (also called "endodomain" or "cytoplasmic signaling domain"). The CAR is sometimes called a "chimeric receptor", a "T-body", or a "chimeric immune receptor (CIR)." The "extracellular domain capable of binding to an antigen" means any oligopeptide or polypeptide that can bind to a certain antigen. In the context of the present invention, the antigen is pTα.

Likewise, the CAR of the invention also comprises a transmembrane domain. The "transmembrane domain" means any oligopeptide or polypeptide known to span the cell membrane, and linking the extracellular and signaling domains. Examples of transmembrane domains include, without limiting to, CD8α transmembrane domain, CD28 transmembrane domain, ICOS transmembrane domain, CD3ξ transmembrane domain, and DAP-12-KIR2DS2 multimeric transmembrane domain. In a particular embodiment of the CAR of the invention, the transmembrane domain is a CD8α transmembrane domain.

The CAR of the invention may comprise a hinge domain between the extracellular domain capable of binding to an antigen and the transmembrane domain. As used herein, a "hinge domain" refers to the short peptide fragment between the scFv and the transmembrane domain that provides conformational freedom to facilitate binding to the target antigen on the tumor cell. It may be used alone or in conjunction with a spacer domain that projects the extracellular domain (e.g. a scFv) away from the T-cell surface. Hinge domains are widely known in the art and any of them can be used in the context of the present invention. Generally, it derives from IgG subclasses (such as IgG1 and IgG4), IgD and CD8α domains, of which IgG1 has been most extensively used. Examples of hinge domains include, without limiting to, segments of immunoglobulins, CD8α, CD28, CD4 or CD3 molecules. In a particular embodiment of the CAR of the invention, the hinge domain is a CD8α hinge domain. As used herein, the term "CD8α hinge domain" refers to a specific protein fragment associated with the lymphocyte surface CD8α antigen. The example sequences of CD8α hinge domain for human, mouse, and other species are widely known in the state of the art. Non-limiting examples of CD8α hinge domains include: human CD8α hinge domain; mouse CD8α hinge domain, and cat CD8α hinge domain. In a particular embodiment of the CAR of the invention, the hinge domain is a human CD8α hinge domain.

The CAR of the invention also comprises, at least, one endodomain. The "endodomain" means any oligopeptide or polypeptide that transmits a signal to cause activation or inhibition of a biological process in a cell. Preferably, the CAR of the invention comprises one, two, or three endodomains, more preferably, the CAR comprises two endodomains. Examples of endodomains include, without limiting to, CD3ξ signaling domain, CD28 coestimulatory domain, 4-1BB (CD137) coestimulatory domain, OX40 coestimulatory domain, ICOS coestimulatory domain, and CD27 coestimulatory domain, alone or in tandem. In a particular embodiment of the CAR of the invention, the endodomain comprises a CD3ξ signaling domain alone or in combination with a CD28 coestimulatory domain or a 4-1 BB coestimulatory domain.

To sum up, in particular embodiment of the CAR of the invention,
- the hinge domain is a IgG4 domain or a CD8α hinge domain, and/or
- the transmembrane domain is a CD28 or a CD8α transmembrane domain; and/or
- the endodomain comprises a CD3ξ signaling domain alone or in combination with a CD28 coestimulatory domain or a 4-1 BB coestimulatory domain.

In a more particular invention of the CAR of the invention
- the hinge domain is a human IgG4 domain or a human CD8α hinge domain, and/or
- the transmembrane domain is a human CD28 or a human CD8α transmembrane domain; and/or
- the endodomain comprises a human CD3ξ signaling domain alone or in combination with a human CD28 coestimulatory domain or a human 4-1BB coestimulatory domain.

In a still more particular embodiment of the CAR of the invention
- the hinge domain is a CD8α hinge domain; and/or
- the transmembrane domain is a CD8α transmembrane domain; and/or
- the endodomain comprises a CD3ξ signaling domain in combination with a 4-1BB coestimulatory domain.

In another still more particular embodiment of the CAR of the invention:
- the hinge domain is a IgG4 domain; and/or
- the transmembrane domain is a CD28 transmembrane domain; and/or
- the endodomain comprises a CD3ξ signaling domain in combination with a CD28 coestimulatory domain.

The present invention also encompasses the nucleotide sequences encoding the antibody, the antigen binding fragment thereof, or the CAR of the invention. Thus, in another aspect, the present invention relates to an isolated nucleotide sequence encoding the antibody or the CAR of the invention as defined in previous paragraphs. Hereinafter, "nucleotide sequence of the invention".

In a particular embodiment of the nucleotide sequence of the invention, the nucleotide sequence encoding the antibody, or the antigen binding fragment thereof, comprises, or consists of, the sequence SEQ ID NO: 9 and/or the sequence SEQ ID NO: 10.
SEQ ID NO: 9: 
GAAGTGCAGTTGGTGGAGTCTGGGGGAGGCTTAGTGAAGCCTGGAGGGTCCC
TGCAACTCTCCTGTGCAGCCTCTGGATTCACTTTCAATAGATATGCCATGTCTT
GGGTTCGCCAGACTCCGGAGAAGAGGCTGGAGTGGGTCGCAGCCATTAGTAG
TGGTGGTAGTTACACCTACTATCCAGACAGTGTGAAGGGTCGATTCATCATCTC
CAGAGACAATGCCAAGAACACCCTGTCCCTGCAAATGAGCAGTCTGAGGTCTG
AGGACACGGCCATGTTTTACTGTGCAAGACTCAGGGTAGTAGCTGAAGGGGGT
AGCTACTTTGACTACTGGGGCCAAGGC ACCACTCTCACAGTCTCCTCA
SEQ ID NO: 10:

GATGTTTTGATGACCCAAACTCCACTCTCCCTGCCTGTCAGTCTTGGAGATCAA
GCCTCCATCTCTTGCAGATCTAGTCAGAGCATTTTACATAGTAATGGAAACACC
TATTTAGAATGGTACCTGCAGAAACCAGGCCAGTCTCCAAAGCTCCTGATCTAC
AAAGTTTCCAACCGATTTTCTGGGGTCCCAGACAGGTTCAGTGGCAGTGGATC
AGGGACAGATTTCACACTCAAGATCAGCAGAGTGGAGGCTGAGGATCTGGGA
GTTTATTACTGCTTTCAAGGTTCACATGTTCCTGTCACGTTCGGCTCGGGGACA
AAGTTAGAAATAAAA

Legend for the sequences: double underlined: CDR1; single underlined: CDR2; dotted underlined: CDR3.

The nucleotide sequence of the invention may comprise other sequences useful in transcription/translation of the antibody, or the antigen binding fragment thereof, such as signal peptide, His tail, stop sequences etc. Thus, in another particular embodiment of the nucleotide sequence of the invention, the nucleotide sequence encoding the antibody of the invention, or the antigen binding fragment thereof, comprises, or consists of, a Kozak consensus sequence, a peptide signal sequence and/or an enhancer sequence, preferably, these sequences are located upstream of the sequence encoding the antigen binding domain of the antibody.

In a more particular embodiment of the nucleotide sequence of the invention, the nucleotide sequence comprises the sequence SEQ ID NO: 11. This nucleotide sequence encodes the peptide comprising, or consisting of, the sequence SEQ ID NO: 12.
SEQ ID NO: 11 
GCCACCATGGGATGGTCCTGTATCATTCTGTTCCTGGTCGCAACTGCCACCGG
GGTCCACTCCGAAGTCCAGCTGGTCGAGAGCGGGGGCGGCCTGGTGAAGCC
AGGAGGCTCCCTGCAGCTGTCTTGCGCCGCCAGCGGCTTCACCTTTAACCGGT
ACGCCATGTCTTGGGTGCGGCAGACACCTGAGAAGAGACTGGAGTGGGTGGC
AGCAATCAGCTCCGGAGGCTCTTATACCTACTATCCAGACAGCGTGAAGGGCA
GGTTCATCATCTCCCGCGATAACGCCAAGAATACCCTGTCCCTGCAGATGTCTA
GCCTGAGATCTGAGGACACAGCCATGTTCTACTGCGCAAGGCTGAGAGTGGTG
GCAGAGGGAGGCAGCTACTTTGATTATTGGGGCCAGGGCACCACACTGACCG
 
TGTCCTCTGGCGGCGGCGGCTCCGGAGGCGGCGGCTCTGGAGGAGGAGGCA
GCGGCGGAGGAGGCTCCGACGTGCTGATGACCCAGACACCACTGTCCCTGCC
CGTGAGCCTGGGCGATCAGGCCTCTATCAGCTGTAGGAGCTCCCAGAGCATC
CTGCACTCCAACGGCAATACATACCTGGAGTGGTATCTGCAGAAGCCAGGCCA
GTCCCCCAAGCTGCTGATCTATAAGGTGTCTAATAGGTTCAGCGGCGTGCCTG
ACCGCTTTTCCGGCTCTGGCAGCGGCACCGATTTCACACTGAAGATCAGCAGG
GTGGAGGCAGAGGACCTGGGCGTGTACTATTGTTTTCAGGGCTCCCACGTGC
CTGTCACCTTCGGCTCTGGAACTAAACTGGAAATCAAACATCACCACCATCACC
ACTAA
SEQ ID NO: 12

ATMGWSCIILFLVATATGVHSEVQLVESGGGLVKPGGSLQLSCAASGFTFNRYAM
SWVRQTPEKRLEWVAAISSGGSYTYYPDSVKGRFIISRDNAKNTLSLQMSSLRSED
TAMFYCARLRVVAEGGSYFDYWGQGTTLTVSSGGGGSGGGGSGGGGSGGGGS
DVLMTQTPLSLPVSLGDQASISCRSSQSILHSNGNTYLEWYLQKPGQSPKLLIYKV
SNRFSGVPDRFSGSGSGTDFTLKISRVEAEDLGVYYCFQGSHVPVTFGSGTKLEIK
HHHHHH*

Legend for the above sequences:
**Bold:** Kozak sequence; *cursive:* signal peptide; single underlined: V_{H} region of scFv; dotted underlined: V_{L} region of scFv; *cursive and single underlined*: His tail; double underlined: stop codon.

As the skilled person in the art understands, the nucleotide sequence of the invention may be introduced in a vector in order to transfer foreign genetic material into a cell and produce the antibody, or the antigen binding fragment thereof, or the CAR of the invention. Thus, in another aspect, the present invention relates to a vector comprising, or consisting of, the isolated nucleotide sequence or the invention, hereinafter "vector of the invention".

As used herein, the term "vector" or "plasmid" refers to any vehicle used to transfer foreign genetic material into a cell. Said vector can be a viral vector or a non-viral vector, such as a plasmid. By way of a non-limiting illustration, said vectors can be viral vectors based on retroviruses, adenoviruses, alphaviruses, etc., or in case of non-viral vectors, the vectors can be DNA-liposome, DNA-polymer, DNA-polymer-liposome complexes, pSilencer 4.1-CMV (Ambion), pcDNA3, pcDNA3.1/hyg pHCMV/Zeo, pCR3.1, pEFI/His, pIND/GS, pRc/HCMV2, pSV40/Zeo2, pTRACER-HCMV, pUB6/V5-His, pVAXI, pZeoSV2, pCI, pSVL and pKSV-10, pBPV-1, pML2d, pTDTI, etc. Said viral and non-viral vectors comprising the polynucleotide encoding in its natural state a fluorescent protein can be administered to the cell by the conventional methods previously cited.

In a particular embodiment of the vector of the invention, the vector is a plasmid, a retroviral vector, a lentiviral vector, an adenoviral vector, and an adeno-associated viral vector.

The vector can contain, among others, multiple cloning sites, expression regulating sequences, suitable replication origins for the host cell in which the vector is introduced, selection markers, etc. In a particular embodiment, the vector is an expression vector which allows the expression of the nucleotide sequence or polynucleotide in the target cell and generally has a promoter sequence (or expression regulating sequences) that drives expression thereof. The promoter sequence preceding the polynucleotide is operatively bound to the said polynucleotide. As used in this description, the expression "operatively bound" means that the polynucleotide is within the correct reading frame for their expression under the control of said regulating sequences. The regulating sequences useful for the present invention can be nuclear promoting sequences or, alternatively, enhancer sequences and/or other regulating sequences increasing the expression of the polynucleotide. The promoter can be constitutive or inducible. If the constant expression of the polynucleotide is desired, then a constitutive promoter is used. Examples of well-known constitutive promoters include the cytomegalovirus (CMV) immediate-early promoter, the Rous sarcoma virus promoter, and the like. A number of other examples of constitutive promoters are well known in the art and can be used in implementing the invention. If the controlled expression of the polynucleotide is desired, then an inducible promoter must be used. In a non-induced state, the inducible promoter must be "silent". "Silent" is understood to mean that in the absence of an inducer, little or no expression of the polynucleotide is detected; in the presence of an inducer, however, the expression of the polynucletide occurs. The expression level can frequently be controlled by varying the concentration of the inducer so that the inducible promoter is stimulated more strongly or weakly and consequently, the concentration of the polynucleotide transcribed product is affected. Examples of well-known inducible promoters are: an androgen or estrogen-responsive promoter, a doxycicline-responsive promoter, a metallothionein promoter, or a promoter responding to ecdysone. Other various examples are well known in the art and can be used for implementing the invention.

In order to express the nucleotide sequence encoding the antibody of the invention, or an antigen biding fragment thereof, o the CAR of the invention, said nucleotide sequence has to be introduced in an expression system such as a host cell. Taking this into account, the nucleotide sequence may be optimized for a proper expression in the chosen host cell. Thus, in another aspect, the present invention relates to a cell, hereinafter "cell of the invention", comprising or consisting of:
- the CAR of the invention; and/or
- the isolated nucleotide sequence of the invention; and/or
- the vector of the invention.

The term "host cell" means any cell type that is susceptible to transformation, transfection, transduction, and the like with the nucleotide sequence or vector of the invention.

The vector introduced into a host cell may be maintained as a chromosomal integrant or as a self-replicating extra-chromosomal vector. The term "host cell" also encompasses any progeny of a parent cell that is not identical to the parent cell due to mutations that occur during replication.

The host cell may be any cell useful in the recombinant production of the antibody, or an antigen binding fragment, or the CAR of the present invention, e.g., a prokaryote or a eukaryote such as a mammalian, insect, plant, or fungal cell. Any method known in the art for introducing DNA into a host cell can be used in the context of the present invention.

The prokaryotic host cell may be any Gram positive bacterium or Gram negative bacterium. Gram positive bacteria include, but not limited to, *Bacillus, Streptococcus, Streptomyces, Staphylococcus, Enterococcus, Lactobacillus, Lactococcus, Clostridium, Geobacillus,* and *Oceanobacillus.* Gram negative bacteria include, but not limited to, *E. coli, Pseudomonas, Salmonella, Campylobacter, Helicobacter, Flavobacterium, Fusobacterium, Neisseria,* and *Ureaplasma.* In a particular embodiment, the host cell is *E. coli.* Examples of *E. coli* strains useful for vector propagation and cloning procedures include, without limiting to, *E. coli* DH10B (Invitrogen), DH5α (ATCC, Invitrogene), DM1 (Invitrogen), GeneHogs (Invitrogen), HB101 (Bio-Rad, Invitrogene, Promega), INV110 (Invitrogen), JM109 (ATCC, Promega), JS5 (Bio-Rad), LE392 (ATCC), NM522 (AS), SCS110 (Stratagene), STBL4 (Invitrogen), SURE (ATCC, Stratagene), TG1 (Stratagene), XL10-Gold (Stratagene) and XL1-Blue MRF (Stratagene).

Examples of insect cells include, without limiting to, *Spodoptera frugiperda* cells (such as SF9 cell or SF21 cell or Sf900+), a BTI-Tn-5B1-4 insect cell from Trichoplusia ni (High-five^{™}, Invitrogen, Carlsbad Calif.), expresSF+^{®}, Drosophila Schneider 2 (S2) Cells, Se301, SelZD2109, SeUCR1, MG-1, Tn368, HzAm1, Ha2302, Hz2E5 and High Five from Invitrogen.

Examples of the fungi host cells includes, without limiting to, Aspergillus (e.g. *Aspergillus awamori, Aspergillus fumigatus, Aspergillus foetidus, Aspergillus japonicus, Aspergillus nidulans, Aspergillus niger* or *Aspergillus oryzae* cell), Aureobasidium, Bjerkandera, Ceriporiopsis (e.g. *Ceriporiopsis aneirina, Ceriporiopsis aneirina, Ceriporiopsis caregiea, Ceriporiopsis gilvescens, Ceriporiopsis pannocinta, Ceriporiopsis rivulosa, Ceriporiopsis subrufa, Ceriporiopsis subvermispora),* Chrysosporium (e.g. *Chrysosporium keratinophilum, Chrysosporium lucknowense, Chrysosporium tropicum, Chrysosporium merdarium, Chrysosporium inops, Chrysosporium pannicola, Chrysosporium queenslandicum, Chrysosporium zonatum),* Coprinus (e.g. *Coprinus cinereus),* Coriolus (e.g. *Coriolus hirsutus),* Cryptococcus, Filibasidium, Fusarium (e.g. *Fusarium bactridioides, Fusarium cerealis, Fusarium crookwellense, Fusarium culmorum, Fusarium graminearum, Fusarium graminum, Fusarium heterosporum, Fusarium negundi, Fusarium oxysporum, Fusarium reticulatum, Fusarium roseum, Fusarium sambucinum, Fusarium sarcochroum, Fusarium sporotrichioides, Fusarium sulphureum, Fusarium torulosum, Fusarium trichothecioides,* or *Fusarium venenatum* cell), Humicola (e.g. *Humicola insolens, Humicola lanuginosa),* Magnaporthe, Mucor (e.g. *Mucor miehei*), Myceliophthora (e.g. *Myceliophthora thermophila),* Neocallimastix, Neurospora (e.g. *Neurospora crassa),* Paecilomyces, Penicillium (e.g. *Penicillium purpurogenum),* Phanerochaete (e.g. *Phanerochaete chrysosporium),* Phlebia (e.g. *Phlebia radiata),* Piromyces, Pleurotus (e.g. *Pleurotus eryngii*), Schizophyllum, Talaromyces, Thermoascus, Thielavia (e.g. *Thielavia terrestris),* Tolypocladium, Trametes (e.g. *Trametes villosa or Trametes versicolor),* or Trichoderma (e.g. *Trichoderma harzianum, Trichoderma koningii, Trichoderma longibrachiatum, Trichoderma reesei, or Trichoderma viride* cell) cell.

The host cell may also be a yeast cell. "Yeast" as used herein includes ascosporogenous yeast (Endomycetales), basidiosporogenous yeast, and yeast belonging to the Fungi Imperfecti (Blastomycetes). More preferably, the yeast host cell is a Candida, Hansenula, Kluyveromyces (e.g. *Kluyveromyces lactis),* Pichia (e.g. *Pichia pastoris),* Saccharomyces (e.g. *Saccharomyces carlsbergensis, Saccharomyces cerevisiae, Saccharomyces diastaticus, Saccharomyces douglasii, Saccharomyces kluyveri, Saccharomyces norbensis, or Saccharomyces oviform is,* etc.), Schizosaccharomyces, or Yarrowia cell *(Yarrowia lipolytica).*

The host cell may also be microalgae. Examples of microalgae include, without limiting to, microalgae from the genera *Chlamydomonas* (e.g. *Chlamydomonas reinhardtii*), *Botryococcus, Neochloris, Nannochloropsis, Phorphyridium, Scenedesmus, Chlorella, Tetraselmis* and *Spirulina.* The chloroplast of these algae are particularly well suited to the production of bacterial proteins as it mimics the native bacterial expression environment of these proteins while being devoid of their cell wall target.

The host cell may also be a plant cell, wherein the vacuolar deposition of recombinant proteins in plant vegetative organs is used to increase yields. To achieve this, the nucleotide sequence of the invention may be fused to sequence specific vacuolar sorting signals (ssVSS) typical of proteases or proteinase inhibitors and/or Ct-VSS representative of storage proteins or plant lectins. Both types of motifs were capable to increase accumulation. Importantly, the type of VSSs or position, either the N or C-terminus, did not alter protein stability, levels or post-translational modifications. Examples of plant cells include, without limiting to, *Nicotiana* sp. (e.g. *N. tabacum, N. benthamiana,* etc.) sugarcane, tomato *(Solanum lycopersicum)* and carrot *(Daucus carota*)*.*

Examples of mammal cells which can be used in the context of the present invention include, without limiting to, a T-cell, a B-cell, an NK cell, epithelial cell lines (porcine, human, etc.), osteosarcoma cell lines (human, etc.), neuroblastoma cell lines (human, etc.), epithelial carcinomas (human, etc.), glial cells (murine, human, etc.), liver cell lines (monkey, etc.), CHO cells (ChineseHamsterOvary), COS cells, BHK cells, HeLa, 911, AT1080, A549, 293 or PER cells. C6, NTERA-2 human ECC cells, D3 cells of the mESC line, human embryonic stem cells such as HS293 and BGV01, SHEF1, SHEF2 and HS181, NIH3T3, 293T, REH and MCF-7 cells and hMSCs (human mesenchymal stem cells), and GliNS2 cells (glioma stem cells). In particular embodiment of the cell of the invention, the cell is a T-cell.

In another aspect, the present invention relates to a composition, hereinafter "composition of the invention", comprising
- the antibody of the invention; and/or
- the CAR of the invention; and/or
- the isolated nucleotide sequence of the invention; and/or
- the vector of the invention; and/or
- the cell of the invention.

According to the conventional techniques known to those skilled in the art, the composition according to the present invention may be formulated with excipient and/or carrier. Thus, in a particular embodiment, the composition of the invention comprises an excipient and/or carrier and/or adjuvant. In case of a therapeutic used of the composition of the invention, the composition is a pharmaceutical composition and it may be formulated with a pharmaceutically acceptable excipient and/or carrier, and/or adjuvant.

The term "excipient" refers to a substance which helps to absorb any of the components of the composition of the invention, stabilizes said components or helps in the preparation of the composition in the sense of giving it consistency or, if necessary, providing flavors which make them more pleasant. Thus, excipients could have the function of keeping the components bound together, such as for example starches, sugars or celluloses, a sweetening function, a colorant function, the function of protecting the medicament, such as for example isolating it from the air and/or moisture, a filler function for a tablet, capsule or any other form of formulation, such as for example dibasic calcium phosphate, a disintegrating function to facilitate the dissolution of the components and their absorption in the intestine, without excluding other types of excipients not mentioned in this paragraph. Therefore, the term "excipient" is defined as any material included in the galenic forms which is added to the active ingredients or to its associations to enable its preparation and stability, modify its organoleptic properties or determine the physical/chemical properties of the composition and its bioavailability. The "pharmaceutically acceptable" excipient should allow for the activity of the compounds of the pharmaceutical composition, that is to say, for it to be compatible with said components. Examples of excipients are agglutinants, fillers, disintegrators, lubricants, coaters, sweeteners, flavorings and colorants. Non-limiting, more specific examples of acceptable excipients are starches, sugars, xylitol, sorbitol, calcium phosphate, steroid fats, talc, silica or glycerin, amongst others.

The term "carrier" refers to a compound which facilitates the incorporation of other compounds to allow a better dosing and administration or to give consistency and form to the composition. Therefore, the carrier is a substance which is used to dilute any of the components of the composition of the present invention to a determined volume or weight, or even without diluting said components, capable of allowing better dosing and administration or giving consistency. When the form of presentation is liquid, the carrier is the diluent. In a particular embodiment, the carrier is pharmaceutically acceptable.

The composition of the present invention can also take the form of a sustained release drug formulation or any other conventional release system, such as, for example, nanoparticles, liposomes or nanospheres, polymeric material, biodegradable or non-biodegradable implant, or biodegradable microparticles, such as, for example, biodegradable microspheres.

The composition of the invention may be employed as a therapeutic agent to be administered to a subject. In this case, the composition provided by this invention is considered a pharmaceutical composition which may be administered by any appropriate administration route; to this end, said composition will be formulated in the suitable form for the selected administration route.

The "galenic form" or "pharmaceutical form" is the arrangement to which the active ingredients and excipients adapt in order to form a medicament. It is defined by the combination of the form in which the pharmaceutical composition is presented by the manufacturer and the form in which it is administered.

Routes of administration include topical, ocular, nasal, pulmonary, buccal, parenteral (intravenous, subcutaneous, and intramuscular), oral, vaginal and rectal. Administration from implants is also possible. The composition of the invention, in particular the pharmaceutical composition, may also be administered topically to the skin or mucosa, that is, dermally or transdermally. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated.

The composition of the invention may also be administered directly into the blood stream, into muscle, or into an internal organ. Suitable means for parenteral administration include intravenous, intra-arterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

The composition of the invention may also be administered intranasally or orally. Dosage forms for oral administration include, without limiting to, tablets (e.g., coated or uncoated tablets), capsules (e.g., soft gelatin capsules, hard gelatin capsules, HPMC capsules, or HPMCP capsules), a capsule inside a capsule, lozenges, troches, ovules, solutions, emulsions, suspensions, syrups, elixirs, powders and granules for reconstitution, dispersible powders and granules, medicated gums, chewing tablets, effervescent tablets, and multiparticulate dosage forms. The pharmaceutical composition may also be provided in bandages or in sutures or the like.

Biodegradable particles (nanoparticles or microparticles) have been shown to be capable of delivering a variety of therapeutic agents including molecules such as polypeptides or proteins.

The composition of the invention may comprise, further to the antibody of the invention; and/or the CAR of the invention; and/or the isolated nucleotide sequence of the invention; and/or the vector of the invention; and/or the cell of the invention, other therapeutic agents such as antibiotics, painkillers, etc., or other therapeutic agents useful in the treatment of relapses of leukemia, preferably relapses of T-cell acute lymphoblastic leukemia (T-ALL), more preferably relapses of T-ALL pre-TCR+.

In another aspect, the present invention relates to a kit, hereinafter "kit of the invention", comprising
- the antibody of the invention; and/or
- the CAR of the invention; and/or
- the isolated nucleotide sequence of the invention; and/or
- the vector of the invention; and/or
- the cell of the invention, and/or
- the composition of the invention.

The term "Kit" as used in the present invention refers to a product containing the different reagents necessary to carry out the method of the invention, which are packaged to enable them to be transported and stored. The kit may further include, without any limitation, buffers, agents to prevent contamination, inhibitors of protein degradation, etc. Suitable materials for packaging the components of the kit include glass, plastic (polyethylene, polypropylene, polycarbonate and the like), bottles, vials, paper, sachets and the like. Additionally, the kits of the invention may contain instructions for simultaneous, sequential or separate use of the different components in the kit. Said instructions may be in the form of printed material or in the form of an electronic medium capable of storing instructions so that they can be read by a subject, such as electronic storage media (magnetic disks, tapes and the like), optical media (CD-ROM, DVD) and the like.

In another aspect, the present invention relates to the antibody, the CAR, the isolated nucleotide sequence, the vector, the cell, the composition and/or the kit of the invention, for use as a medicament, particularly, for use in the treatment and/or prevention of leukemia, preferably T-cell acute lymphoblastic leukemia (T-ALL), more preferably T-ALL pre-TCR+, in a subject.

The term "medicament", as it is used herein, refers to any substance or combination of substances which has properties for the treatment or prevention of diseases in organisms, preferably in human beings, or which may be used or administered to organisms, preferably to human beings, in order to restore, correct, or modify physiological functions by exerting a pharmacological, immunological, or metabolic effect. The use of the antibody of the invention as a medicament can be carried out alone with the antibody or in combination with other medicaments or compositions by way of a combined therapy, and can be administered simultaneously or at different time intervals (sequentially).

The term "treating" (or "treat" or "treatment") refers to processes involving a slowing, interrupting, arresting, controlling, stopping, reducing, or reversing the progression or severity of an existing symptom, disorder, condition, or disease, but does not necessarily involve a total elimination of all disease-related symptoms, conditions, or disorders. The treatment of a disorder or disease may, for example, lead to a halt in the progression of the disorder or disease (e.g., no deterioration of symptoms) or a delay in the progression of the disorder or disease (in case the halt in progression is of a transient nature only). The "treatment" of a disorder or disease may also lead to a partial response (e.g., amelioration of symptoms) or complete response (e.g., disappearance of symptoms) of the subject/patient suffering from the disorder or disease. It is to be understood that a subject/patient may experience a broad range of responses to a treatment (such as the exemplary responses as described herein above).

The term "prevention", as it is used herein, consists of preventing the onset or reappearance of the disease, that is, preventing the disease or disease state or relapse of the disease in a subject (preferably a mammal, and more preferably a human), particularly, when said subject is predisposed to the pathological condition but has not yet been diagnosed.

The term "leukemia", as it is used herein, refers to the accepted medical term for a cancer of the blood-forming tissues of the bone marrow. It is characterized by the growth of immature white blood cells. The disease can be classified based on which blood cells are abnormal. Lymphomas are cancers of the lymphoid tissue (lymph nodes, spleen, and other organs of the immune system).

The term "acute lymphoblastic leukemia (ALL)", as it is used herein, refers to a hematologic cancer characterized by oncogenic transformation of lymphoid progenitors during their development.

The expression "T-cell acute lymphoblastic leukemia", as it is used herein, refers to a hematologic cancer characterized by abnormal growth of T-cell progenitors (a type of white blood cell - leukocytes) which originate in the thymus and progress rapidly. The expression "pre-TCR+ leukemia-initiating cells", as it is used herein, refers to leukemia-initiating T-cells which express the pre-TCR receptor on their cell surface.

In another particular embodiment of the use of the invention, the antibody, the CAR, the isolated nucleotide sequence, the vector, the cell, the composition and/or the kit of the invention is used in the diagnosis, treatment, and/or prevention of relapses of leukemia, preferably relapses of T-cell acute lymphoblastic leukemia (T-ALL), more preferably relapses of T-ALL pre-TCR+.

The term "relapse", as it is used herein, refers to leukemia which had shown a regression or plateau after treatment, resumes its development or progression, or develops metastases.

The administration of the antibody of the invention or of the pharmaceutical composition of the invention will be carried out in a therapeutically effective dose which is sufficient to demonstrate a benefit for the patient, preferably in relation to a decrease in the number of LICs, more preferably T-ALL LICs, or in the replicative capacity of LICs in the patient. Such benefit may involve improvement of at least one symptom related to acute lymphoblastic leukemia. Prescription of the treatment, that is, decisions about dosages, frequency, duration, etc., will fall under the responsibility of the general practitioner or specialist who cares for the affected patient.

The term "therapeutically effective dose" or "therapeutically effective amount", as it is used in the present invention, refers to an amount (or amount per unit mass of the individual to whom the dose is administered) of a drug or therapeutic agent which causes a detectable therapeutic effect in an individual or a group of individuals to whom the dose is administered, causing minimal side or toxic effects. The therapeutically effective amount useful for producing a therapeutic effect can be determined by means of conventional techniques well known to those skilled in the art. The term "therapeutically effective dose-50" or "therapeutically effective dose-95" includes a statistical value in which the therapeutic effect must be detectable in 50% or 95% of the individuals to whom the dose is administered. With respect to the toxic effects of the drug or compound, it is preferable that the effective therapeutic dose does not cause any toxic effect. However, although toxic effects may occur in some cases, a compromise may be reached in which these effects are considered to be preferable to the normal development of the disease or sickness without administration of the drug or compound, and can in turn be treated by means of additional therapies.

The term "subject" in the context of the present invention is a mammal, e.g., a primate, preferably a higher primate, e.g., a human.

As used herein, "isolated" will mean material removed from its original environment (e.g., the natural environment in which the material occurs), and thus it is "altered by the hand of man" from its natural environment.

In another aspect, the present invention relates to a method of producing anti-pTα CAR T-cells, hereinafter "method of the invention", comprising:
(i) introducing a nucleotide sequence encoding the CAR of the invention in a T-cell or in a population of T-cells; and
(ii) selecting a subpopulation of T-cells that have been successfully transduced with said nucleotide sequence of step (i) thereby producing anti-pTα CAR T-cells.

The term "anti-pTα CAR T-cell" refers to a T-cell that expresses in its surface a CAR which specifically recognizes pTα. In order to obtain said anti-pTα CAR T-cell the method of the invention comprises a first step comprising introducing a nucleotide sequence encoding the CAR of the invention in a T-cell or in a population of T-cells.

Methods for introducing nucleotide sequences into a cell are widely known in the state of the art, and any of them may be used to put into practice the method of the invention.

Next, the cells which have been successfully transduced with the nucleotide sequence of the invention are selected. Example of an assay for selecting the T-cells which express on its surface the CAR of the invention is disclosed in examples of the present description.

In another aspect, the present invention also relates to an *in vitro* method for detecting a leukemia cell in a sample isolated from a subject, hereinafter "the *in vitro* method of the invention", comprising
(a) analysing
   - the levels of pTa in the biological sample by detecting the complex formed by the antibody of the invention, or by the antigen binding fragment thereof, and pTα; or alternatively
   - the proportion of cells in a biological sample showing on the surface (i) the complex formed by the CAR of the invention, or the antigen binding fragment thereof, and pTα, or (ii) the complex formed by the T-cells which express on its surface the CAR of the invention and pTα,
   and
(b) comparing
   - the levels of pTa observed in step (a) with the levels of pTa observed in a control biological sample; or
   - the proportions of pre-TCR+ cells observed in step (a) with the proportions of pre-TCR+ cells observed in a control biological sample,
   wherein the leukemia cell is detected when
   - the level of pTa is elevated compared to that observed in the control biological sample, or
   - the proportion of pre-TCR+ cells is elevated compared to that observed in the control biological sample;
   and
   wherein the sample isolated from a subject is not from thymus.

As use herein, the term "sample" refers to a small part or quantity of something intended to show what the whole is like. In the *in vitro* method of the invention, the sample isolated from a subject is not from thymus. Examples of other biological samples include, without limiting to, cerebrospinal fluid (CSF), bone marrow, saliva, blood, plasma, urine or faeces. The biological sample may come from a subject, both non-human animal and human.

The *in vitro* method of the invention comprises a first step relating to analyze
- the levels of pTa in the biological sample by detecting the complex formed by the antibody, or by the antigen binding fragment thereof, and pTα; or alternatively
- the proportion of cells in a biological sample expressing on the surface (i) the complex formed by the CAR of the invention, or the antigen binding fragment thereof, and pTα, or (ii) the complex formed by the T-cells which express on its surface the CAR of the invention and pTα.

In the context of the present invention, the expression "to analyze the levels of pTα" refers to quantify the amount of pTa present in the biological sample. Likewise, the expressions "to analyze the proportion of cells expressing on the surface the complex formed by the CAR of the invention, and pTα" or "to analyze the proportion of cells expressing on the surface the complex formed by the T-cells which express on its surface the CAR of the invention and pTα" refer to quantify the amount of cells in the biological sample which shows in their surface the complex form by the CAR of the invention, or the T-cell expressing the CAR of the invention, and pTα, i.e. to quantify the amount of pre-TCR+ cells. Methods for quantifying the amount of pTa or the amount of cells showing the mentioned complexes on their surface are widely known in the state of the art.
After step (a), the *in vitro* method of the invention comprises step (b), i.e. comparing the levels of pTa or the proportions of pre-TCR+ cells detected with the corresponding ones in a control biological sample.

As use herein, the term "control biological sample" refers to the biological sample obtained from a subject who does not suffer from leukemia.

Finally, after carrying out step (b), it can be concluded that a leukemia cell is detected when
- the level of pTa is elevated compared to that observed in the control biological sample, or
- the proportion of pre-TCR+ cells is elevated compared to that observed in the control biological sample.

As the skilled person understands, if leukemia cells are detected in a biological sample of a subject, then the subject suffers from leukemia, specifically, particularly, T-cell acute lymphoblastic leukemia (T-ALL), more particularly T-ALL pre-TCR+. Thus, the present invention may also be used as a diagnosing tool.

### DESCRIPTION OF THE DRAWINGS

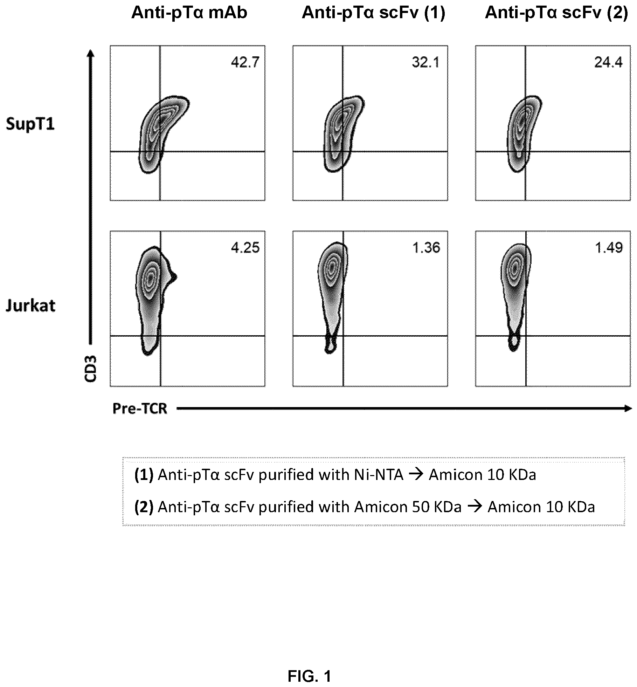

**Figure 1****.** Validation of anti-pTα scFv specificity. Surface pre-TCR receptor expression detected on the SupT1 human T-ALL cell line by the anti-pTα scFv products (middle and right panels) purified either by (1) Ni-NTA chromatography or by (2) Amicon ultra centrifugal filters 50 KDa and subsequently concentrated by Amicon ultra centrifugal filters 10 KDa, in comparison with detection of expression by the anti-pTa mAb (left panels). Bound anti-pTα mAb was detected by flow cytometry with an anti-human IgG (H+L)-FITC secondary Ab, and anti-pTα scFv was detected by incubation with a mouse anti-Histidine mAb followed by an anti-mouse IgG (H+L)-FITC secondary Ab. CD3 expression was detected with an anti-CD3-PE mAb. Jurkat cells (TCRαβ+) were used as a negative control.

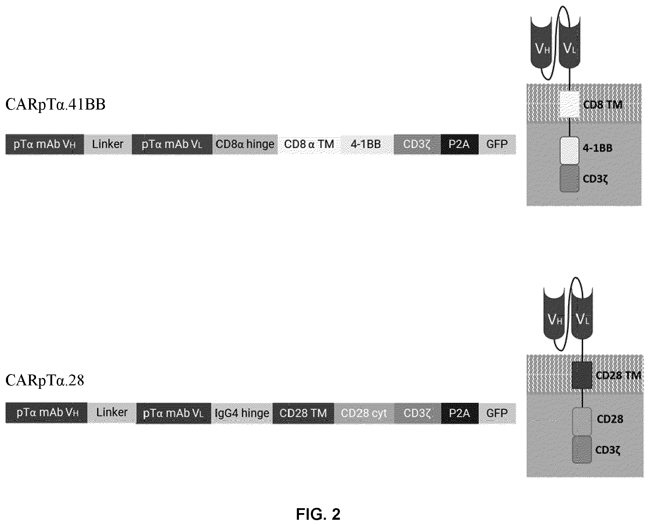

**Figure 2****.** Design and engineering of 2 different anti-pTα-specific 2^{nd} generation CAR constructs. Illustrations of the structure of the different CARpTa constructs generated. The anti-human pTa mAb was used to obtain a specific single chain variable fragment (scFv) composed of the Heavy and Light chains variable regions (V_{H} and V_{L}, respectively) linked through a (Gly4Ser)4 linker. This anti-pTα scFv was cloned into two different second-generation CAR backbones: 1) CARpTα.41BB consisting of human CD8α hinge and transmembrane (TM) domains, and human 4-1BB and CD3ζ endodomains; 2) CARpTα.28 containing human IgG4 hinge, human CD28 TM domain and human CD28 and CD3ζ endodomains. Both constructs were followed by a P2A-GFP cassette.

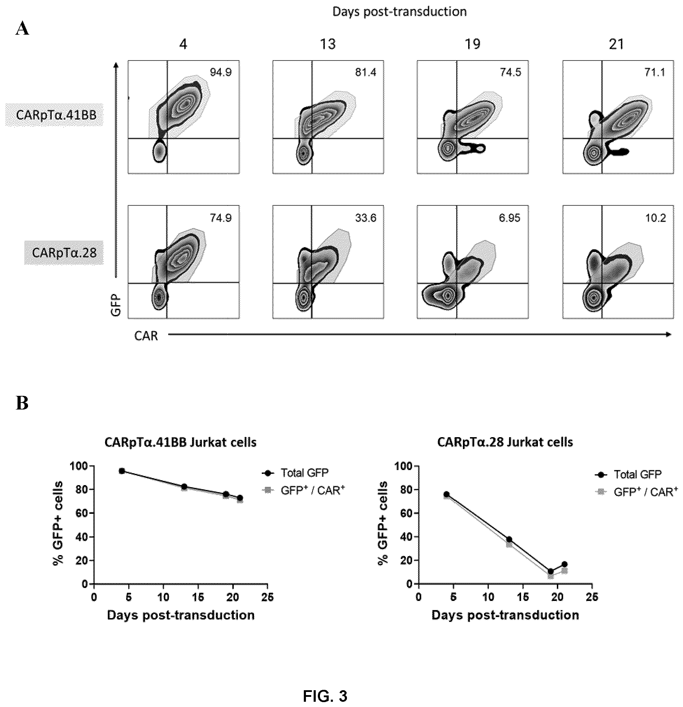

**Figure 3****.** Generation of Jurkat T cells expressing two different CARpTa CAR constructs and monitoring of CARpTa surface expression over time. (A) Flow cytometry analysis at the indicated days post-transduction of Jurkat cells infected with lentiviral (LV) particles encoding either CARpTα.41BB or CARpTα.28 constructs together with GFP. Quantification of transduced cells was assessed by means of GFP expression. Background staining was determined on non-transduced cells (B) Analysis of total transduced (GFP+) and CAR-expressing (GFP+/CAR+) Jurkat cells shown in (A). Data from a representative experiment out of three are shown in percentages of positive cells at the indicated days pos-transduction.

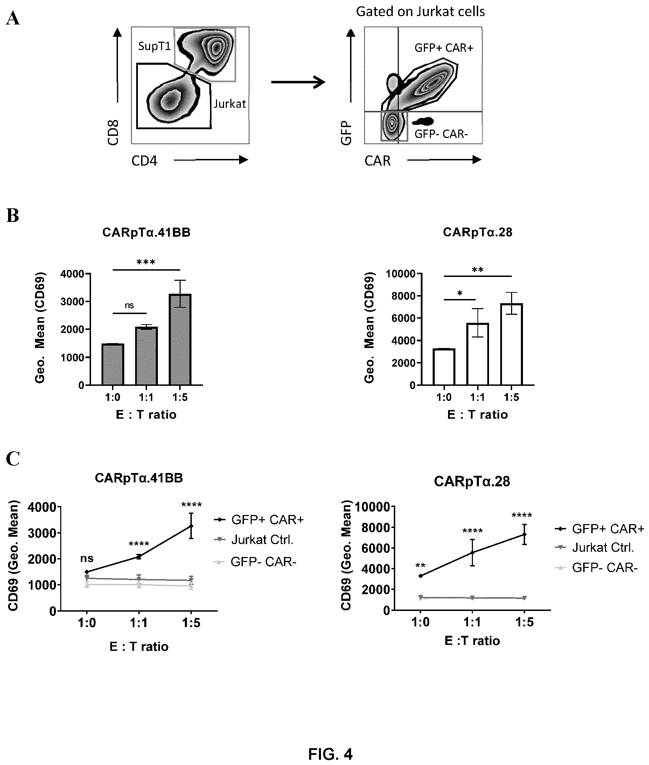

**Figure 4****.** Functional comparison of CARpTα.41BB- and CARpTα.28-armed Jurkat T cells. **(A)** Gating strategy based on CD4 and CD8 expression levels allowing independent flow cytometry analysis of both Jurkat T cells transduced with either CARpTα.41BB or CARpTα.28, and pre-TCR+ SupT1 target cells, co-cultured for 16h (left). GFP and surface CAR expression levels in gated Jurkat cells is shown on the right. **(B)** Flow cytometry analysis of CD69 expression in the GFP+CAR+ population of either CARpTα.41BB- or CARpTα.28-transduced Jurkat cells incubated for 16h either alone (1:0) or with pre-TCR+ SupT1 target cells at increasing effector: target cell ratios (1:1 and 1:5). **(C)** CD69 expression on control Jurkat cells, GFP-CAR-untransduced Jurkat cells and GFP+CAR+ Jurkat cells transduced with either CARpTα.41BB- or CARpTα.28, incubated for 16h either in the absence (1:0) or in the presence of pre-TCR+ SupT1 target cells at 1:1 and 1:5 E:T ratio. Data are shown as means +/- SD of Geometric Mean (Geo Mean) values. (n=3). *P < 0.05, **P < 0.01, ****P < 0.0001. ns, not significant.

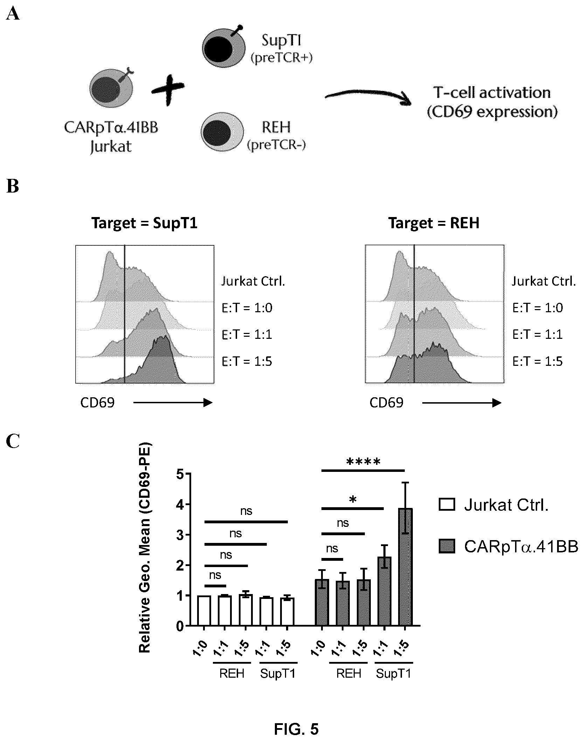

**Figure 5****.** Functional validation of of anti-pTα specificity of CARpTα.41BB expressed on the surface of Jurkat T cells. **(A)** Schematic representation of the pre-TCR-specific T-cell activation experimental assay. (B) Representative flow cytometry histograms of CD69 expressed on CARpTα.41BB-transduced Jurkat cells cultured either in the absence (1:0) or in the presence (1:1 and 1:5 E:T cell ratios) of either pre-TCR+ SupT1 cells or pre-TCR- REH cells. Jurkat cells cultured alone were used as a negative control. **(C)** Relative CD69 expression induced in Jurkat cells by CARpTα41BB-mediated specific activation upon culture with pre-TCR+ SupT1 cells at different E:T cell ratios, but not in the presence of pre-TCR- REH cells. Control Jurkat cells where used as a negative control (n=4). Data are shown as means +/- SD of Geo Mean values. *P < 0.05, **P < 0.01, ***P < 0.001, ****P < 0.0001. ns, not significant.

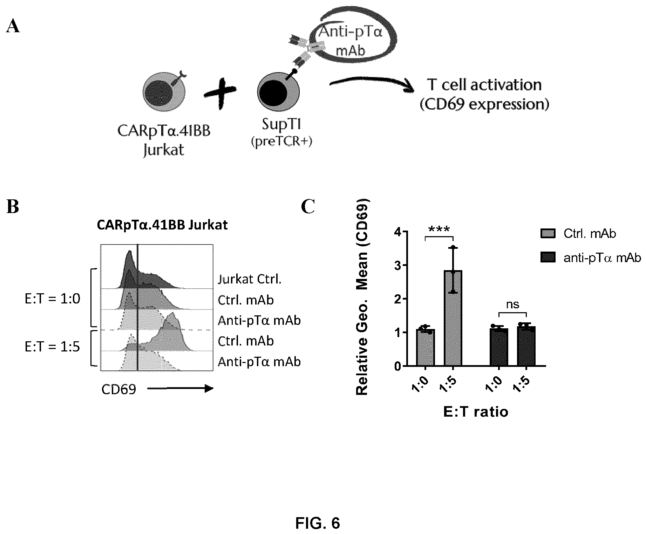

**Figure 6****.** Blocking the CARpTα/pre-TCR interaction with the anti-pTα mAb impairs the activation of CARpTα.41BB-transduced Jurkat T cells. **(A)** Schematic representation of the experimental assay. Target SupT1 cells were pre-incubated with the anti-pTα mAb or with an isotype-matched control mAb (Ctrl. mAb). **(B)** CD69 expression on CARpTα.41BB-transduced Jurkat cells incubated for 16 h either alone (1:0) or with pre-TCR+ SupT1 cells at the indicated effector:target (E:T) cell ratios. Jurkat cells were assayed in parallel as a control of CD69 basal expression levels. (C) CD69 expression on CARpTα.41BB-transduced Jurkat cells cultured with or without pre-TCR+ SupT1 cells that were pre-incubated either with the anti-pTα mAb or with a Ctrl. mAb (n=3). Data are shown as means +/- SD Geo Mean values relative to values observed in Jurkat cells cultured the absence of SupT1 target cells. ***P < 0.001. ns, not significant.

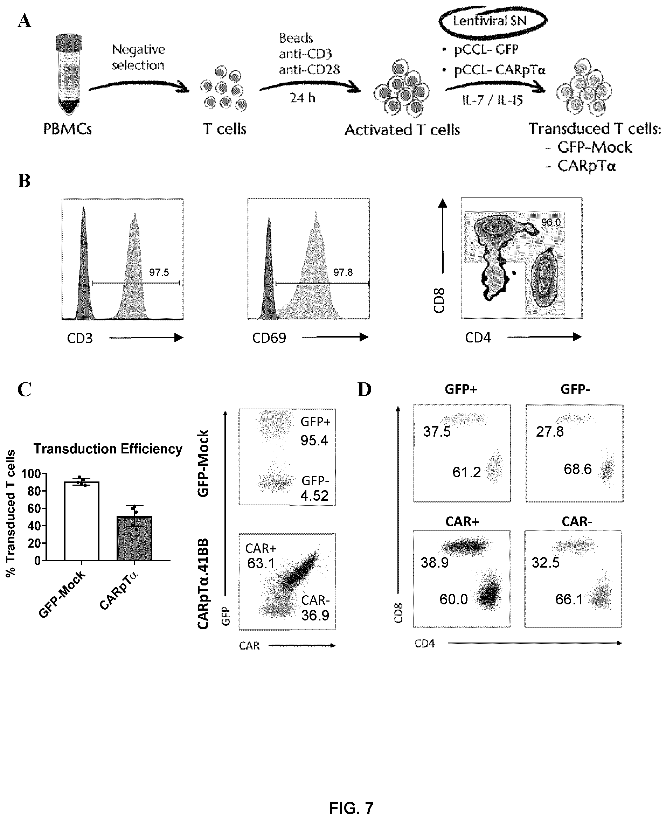

**Figure 7****.** Efficient transduction of primary human T cells with CARpTα.41BB. **(A)** Schematic representation of T-cell isolation from peripheral blood mononuclear cells (PBMC), activation and lentiviral transduction protocol. **(B)** Representative phenotype of activated T cells treated with anti-CD3/CD28-coupled beads for 24 h, and evaluated by flow cytometry for CD3, CD69, CD4 and CD8 expression. Isotype-matched irrelevant mAbs were used as negative controls (n = 4). **(C)** Transduction efficiencies of GFP-Mock- and CARpTα.41BB-transduced human primary T cells (n = 5). **(D)** CD4 vs CD8 expression in GFP-Mock- and CARpTα.41BB-transduced (GFP+) T cells compared to non-transduced (GFP-) T cells, electronically gated based on GFP and CAR expression as shown in the biparametric histogram shown on the left (n = 4).

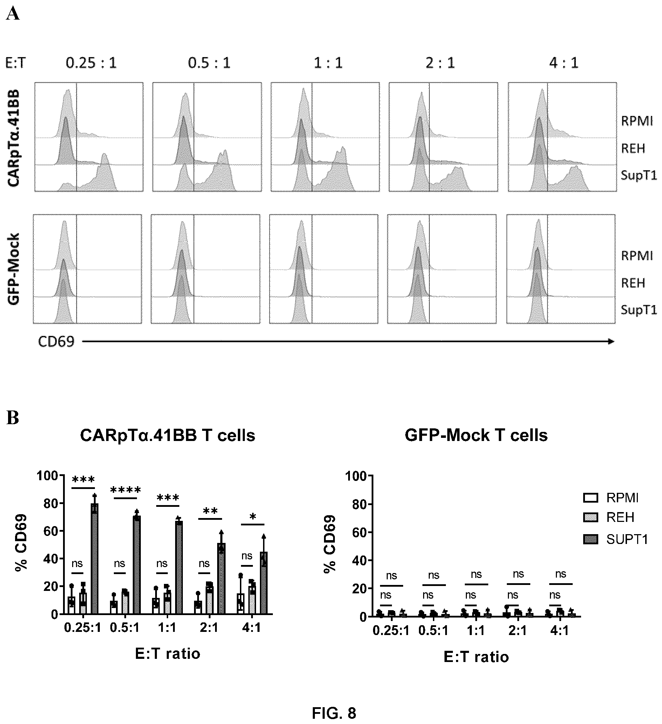

**Figure 8****.** Specific activation of CARpTα.41BB-transduced primary human T cells by recognition of the pre-TCR+ SupT1 cell line **(A)** Flow cytometry analysis of T-cell activation measured by means of induction of CD69 expression on primary T cells either transduced with the CARpTα.41 BB or GFP-Mock-transduced, upon culture with pre-TCR+ (SupT1) or pre-TCR- (REH) cells at the indicated effector:target (E:T) cell ratios, or upon incubation in culture medium in the absence of target cells (RPMI). Representative results of one out of three experiments is shown **(B)** Percentages of activated primary T cells either transduced with the CARpTα.41BB (left) or GFP-Mock-transduced (right), measured by means of induction of CD69 expression upon co-culture with REH or SupT1 target cell lines at the indicated E:T cell ratios or upon culture in medium with no target cells. Data are shown as means +/- SD. (n=3). *P < 0.05, **P < 0.01, ***P < 0.001, ****P < 0.0001. ns, not significant.

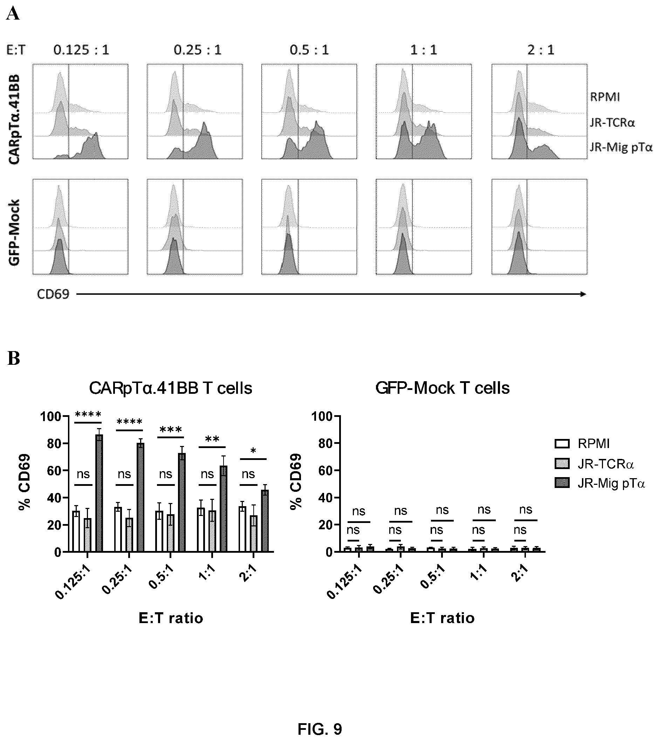

**Figure 9****.** CARpTα.41BB-transduced primary human T cells specifically become activated by recognition of the human pTα chain of the pre-TCR. **(A)** Representative flow cytometry analysis of T-cell activation measured by means of induction of CD69 expression on primary T cells either transduced with the CARpTα.41 BB or GFP-Mock-transduced, upon culture with the pTα-transduced JR cell line (JR-Mig pTα, pre-TCR+) or with the JR cell line transduced with TCRα (JR-TCRα, TCRαβ+ pre-TCR-) cell line at different effector:target (E:T) cell ratios, or cultured in the absence of target cells (RPMI). **(B)** Percentages of activated primary T cells either transduced with the CARpTα.41BB (left) or GFP-Mock-transduced (right), measured by means of induction of CD69 expression upon co-culture with JR-Mig pTa or JR-TCRα target cell lines at different E:T cell ratios or upon incubation in RPMI medium with no target cells. Data are shown as means +/- SD. (n=3). *P < 0.05, **P < 0.01, ***P <0 .001, ****P < 0.0001. ns, not significant.

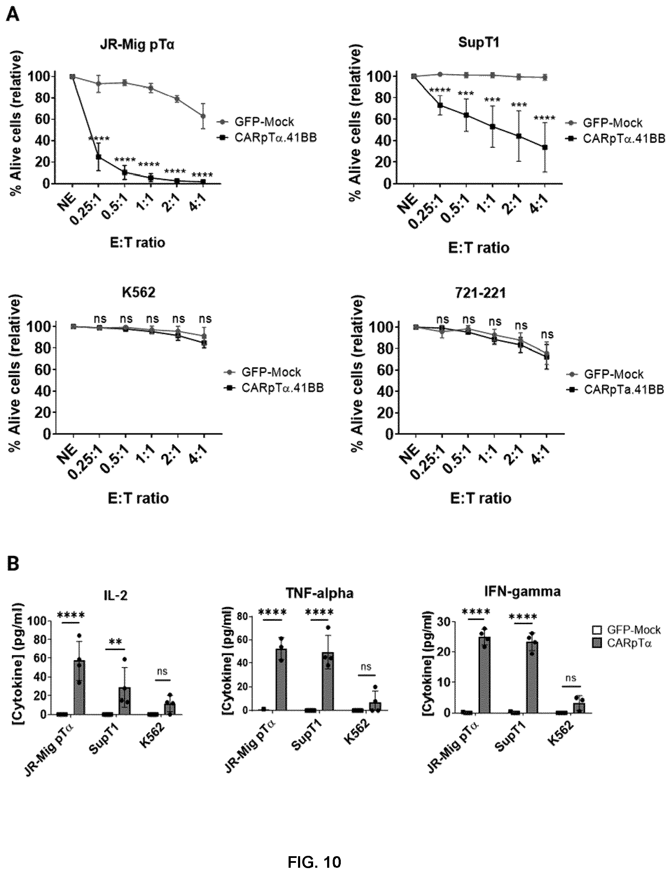

**Figure 10****.** Specific acquisition of cytotoxic potential and induction of pro-inflammatory cytokine production by CARpTα.41BB-transduced human primary T cells upon pre-TCR recognition. **(A)** Either pre-TCR+ (JR-Mig-pTα and SupT1) or pre-TCR- (K562 and 721-221) target cells were labelled with CellTrace^{™} Violet and incubated with GFP-Mock- or CARpTα.41 BB-transduced T cells at the indicated effector: target (E:T) cell ratios for 16 h. CARpTα.41BB-mediated cytotoxicity was determined by flow cytometry by quantification of alive (7-amino actinomycin D negative) CellTrace^{™} Violet-positive target cells remaining in each culture condition, relative to cell numbers recovered in cultures set up in the absence of effector cells (NE). Data are shown as mean values +/- SD (n = 6). **(B)** Enzyme-linked immunosorbent assay (ELISA) showing production of the pro-inflammatory cytokines IL-2, IFN-γ and TNFα by CARpTα.41 BB-transduced, but not GFP-Mock-transduced primary T cells co-cultured with pre-TCR+ JR-Mig-pTα and SupT1 or pre-TCR- K562 cell lines at an 1:1 E:T cell ratio for 16 h. Data are shown as mean values (pg/ml) +/- SD. (n=4). *P < 0.05, **P < 0.01, ***P < 0.001, ****P < 0.0001. ns, not significant.

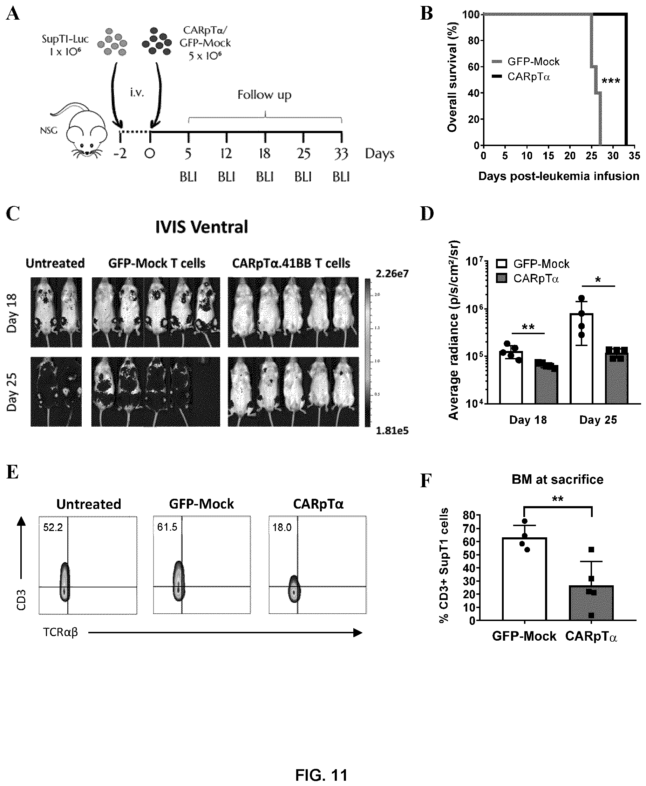

**Figure 11****.** CARpTa-T cell therapy hampers SupT1 leukemia progression and increases overall survival in a mouse preclinical model. **(A)** Scheme of the CARpTa-T cell therapy in NSG immunodeficient mice injected with luciferase-transduced SupT1 pre-TCR+ leukemic cells (SupT1-Luc) and treated with CARpTa-transduced primary human T cells. **(B)** Overall survival of NSG mice injected with SupT1-Luc preTCR+ cells and receiving either CARpTa-transduced or GFP-Mock-transduced T cells as indicated in (A). Two-tailed Mann-Whitney t-test was used. **(C)** Ventral IVIS imaging of tumor burden monitored by bioluminescence imaging (BLI) in mice in (B) at the indicated time points. Mice not receiving human T cells were included as control. **(D)** Average photon count of ventral and dorsal acquisitions per animal at days 18 and 25 post-CAR-T cell infusion. **(E)** Representative flow cytometry analysis of the phenotype of leukemic cells recovered from the BM of mice treated with either GFP-Mock- or CARpTa-transduced T cells at the end point. **(F)** Analysis of CD3 expression on SupT1 leukemic cells remaining in the BM of mice treated with GFP-Mock- or CARpTa-transduced T cells (n = 5 mice per group). Data are shown as mean +/- SD percentages of CD3+ cells. *P < 0.05, **P < 0.01, ***P < 0.001, ****P < 0.0001. ns, not significant. (i.v: intravenous injenction).

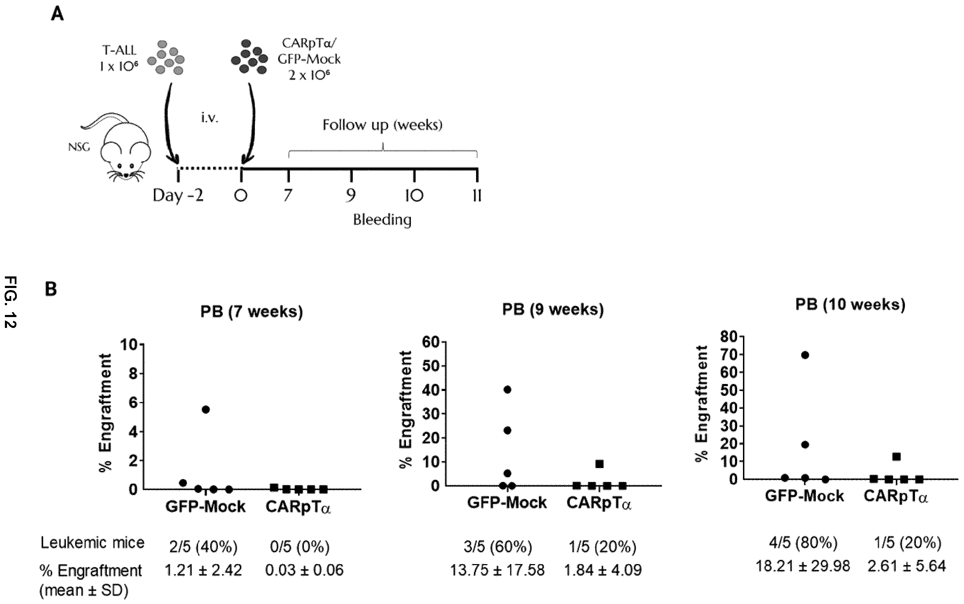

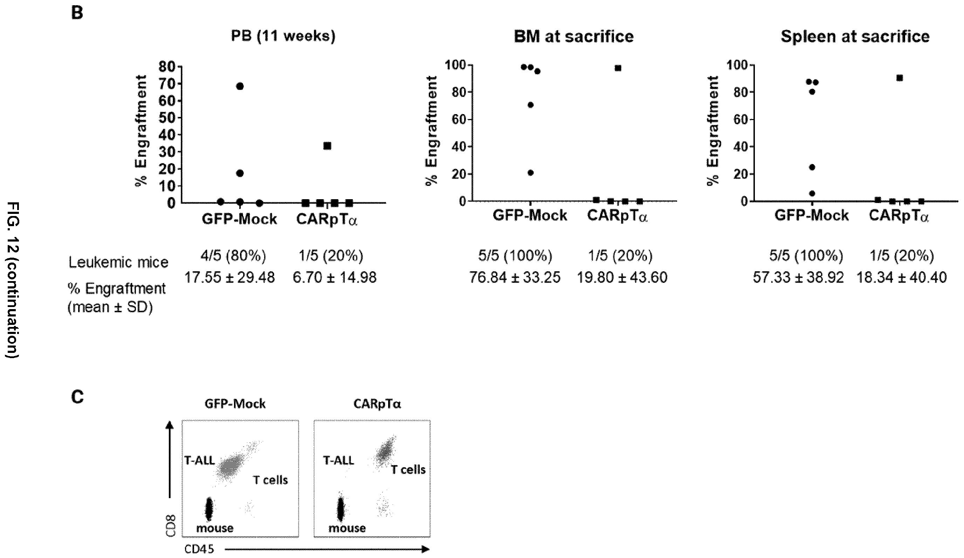

**Figure 12****.** CARpTa-T cell therapy hampers progression of primary human T-ALL blasts in a patient-derived xenograft (PDX) mouse model. **(A)** Scheme of the PDX *in vivo* model. **(B)** Leukemia engraftment in peripheral blood (PB), bone marrow (BM) and spleen of mice infused with SupT1 cells, and analyzed at the indicated times following administration of human primary T cells transduced with either GFP-Mock-or CARpTα-41BB. Results are shown as percentages of leukemic SupT1 cells per mouse. Numbers and frequencies of mice showing leukemia engraftment in the different plots are also given.

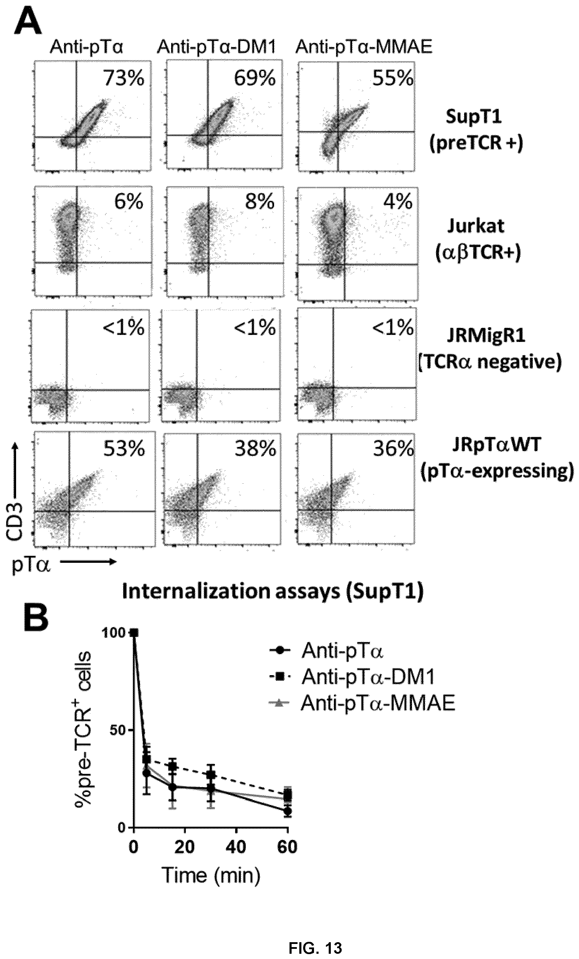

**Figure 13****.** Anti-human pTα antibody-drug conjugates (Anti-pTα-ADC) bind the pre-TCR complex inducing its internalization in pre-TCR-expressing cells. **(A)** Flow cytometry analysis of cell surface pre-TCR expression levels on the indicated cell lines as assessed by labeling with either an anti-human pTα (anti-pTα) monoclonal antibody (mAb) or the anti-pTα mAb conjugated with either DM1 or MMAE drugs. **(B)** Pre-TCR internalization assay of SupT1 cells. Percentages of pre-TCR positive SupT1 cells measured by flow cytometry from triplicate samples upon incubation with the indicated mAb and labeling with a secondary anti-mouse FITC-coupled IgG antibody are shown.

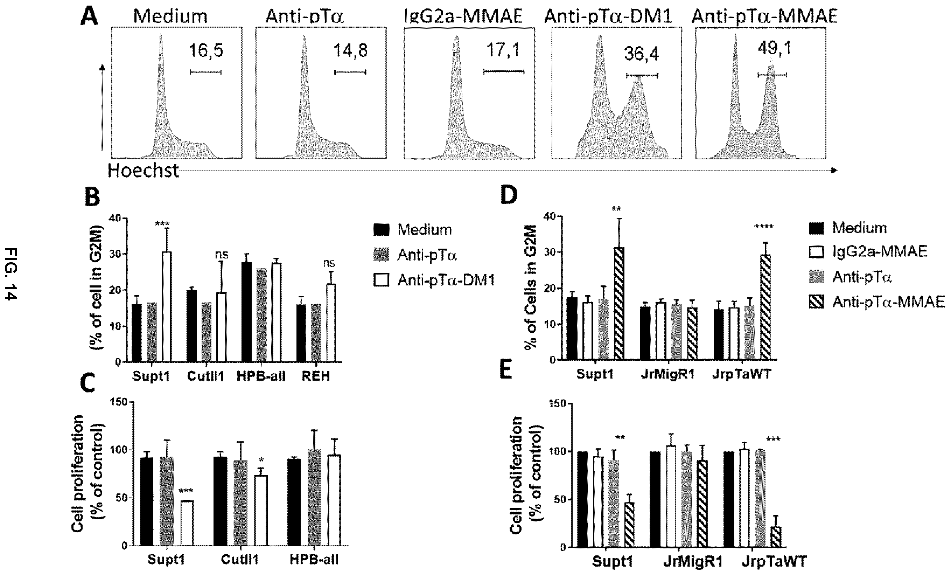

**Figure 14****.** Anti-pTα ADC impairs cell cycle progression and cell proliferation of pre-TCR+ cells. **(A)** Representative cell cycle profiles of SupT1 leukemic cells treated with either the anti-pTα mAb or the ADC derivate. (B, D) Percentages of cells in the G2/M phases of the cell cycle determined by Hoechst labeling and flow cytometry of cells electronically gated as in (A) after 4 days of culture. (C, E) Relative numbers of the indicated leukemic cells after in vitro culture with or without anti-pTα mAb or anti-pTα-DM1 ADC. Data are shown as mean ± SEM of cell numbers recovered from cultures supplemented with anti-pTα mAb and ADCs relative to cell numbers recovered from cultures set up in the absence of antibodies. A two-tailed unpaired t test was used to assess the confidence intervals. (n=3). P < 0.05, **P < 0.01, ***P < 0.001, ****P < 0.0001.

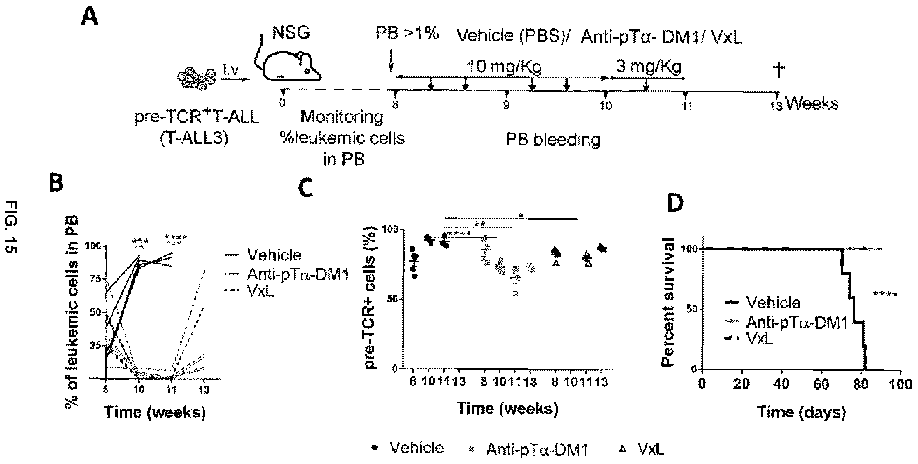

**Figure 15****.** Pre-TCR targeting by anti-pTα-DM1 impairs progression of patient-derived primary T-ALL cells and increases overall survival in an *in vivo* preclinical assay (A) Schematic representation of a T-ALL therapy strategy based on administration of anti-pTα-DM1 ADC in a T-ALL (T-ALL3) patient-derived PDX mouse model. Percentages of total (B) and pre-TCR-expressing (C) leukemic cells in the peripheral blood (PB) of mice transplanted with patient-derived primary T-ALL cells and treated with vehicle (PBS) or with DM1-conjugated anti-pTα mAb (Anti- pTα-DM1 ADC) or with VxL (D) Kaplan-Meier survival curves of T-ALL transplanted mice receiving either anti-pTα ADCs or control vehicle. Data are shown as mean ± SEM percentages of values from 5 mice per group. A two-tailed unpaired t test was used to assess the confidence intervals

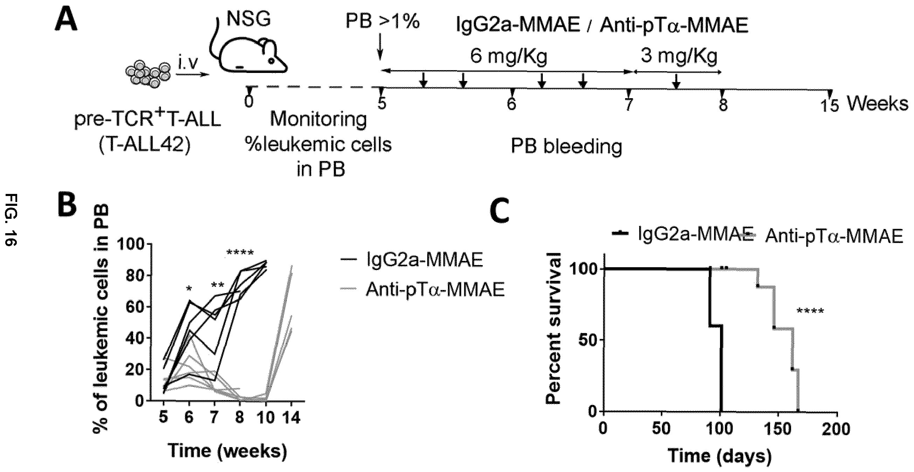

**Figure 16****.** Pre-TCR targeting by anti-pTα-MMAE impairs progression of patient-derived primary T-ALL cells and increases overall survival in an *in vivo* preclinical assay. (A) Schematic representation of a T-ALL therapy strategy based on administration of anti-pTα-MMAE ADC in a T-ALL (T-ALL42) patient-derived PDX mouse model. (B) Percentages of total leukemic cells in the peripheral blood (PB) of mice transplanted with patient-derived primary T-ALL cells and treated with either isotype-matched control ADC (IgG2a-MMAE) or anti-pTα-MMAE ADC (C) Kaplan-Meier survival curves of mice transplanted with T-ALL patient cells and treated with either anti-pTα-MMAE ADC or control ADC (IgG2a-MMAE). Data are shown as mean ± SEM percentagesderived from 5 mice per group. A two-tailed unpaired t test was used to assess the confidence intervals.

### Examples

### MATERIAL AND METHODS

### 1. Molecular cloning and sequencing

### 1.1. Design and synthesis of the anti-pTα scFv coding sequence

The coding sequence for the anti-pTα scFv construct was commercially synthetised (GenScript). The construct comprised the following elements:
1) a Kozak sequence;
2) a signal peptide or leader sequence;
3) the V_{H} and V_{L} regions joined through a (GlySer₄)₄ linker; wherein the V_{H} region comprises the nucleotide sequence SEQ ID NO: 9 and the V_{L} region comprises the nucleotide sequence SEQ ID NO: 10;
4) a polyhistidine tag (His); and
5) a stop codon.

This anti-pTα scFv construct shows the nucleotide sequence SEQ ID NO: 11, and it was cloned by restriction with Hindlll and BamHl enzymes into the pcDNA 3.1 expression vector (pcDNA 3.1-scFv). To allow cell tracing by GFP expression, an IRES-GFP cassette from the pHRSIN-IRES-GFP lentiviral vector (González-García et al., 2019 Blood, 134:2171-2182) was then cloned 3' of the His tag by enzymatic digestion with BamHl and Notl and subsequent ligation (pcDNA 3.1-scFv-IRES-GFP). The amino acid sequence coded by the anti-pTα scFv construct (SEQ ID NO: 11) comprises the sequence SEQ ID NO: 12.

### 1.2. Generation of CARpTα constructs

Two different CARpTa constructs were generated by means of Gibson assembly (New England Biolabs, NEB), where the anti-pTα scFv above generated was cloned into two different second-generation CAR backbones, together with a P2A-GFP cassette. The two different CAR backbones are detailed below (See Figure 2):
1) **CAR.41BB** backbone consisted of a human CD8α hinge, human CD8α transmembrane (TM) domain, and human 4-1BB and CD3ζ endodomains.
2) **CAR.28** backbone was composed of a human IgG4 hinge (with extra spacer IgG1 CH3), human CD28 TM domain, and human CD28 and CD3ζ endodomains.

Both complete CARpTα-P2A-GFP constructs were finally cloned into the pCCL lentiviral (LV) vector by enzyme digestion and subsequent ligation. Identical LV vector expressing GFP alone (GFP-Mock vector) was used as a control.

### 2. Human peripheral blood T-cell isolation

Human peripheral blood samples (buffy coat) from healthy donors were obtained under a collaboration agreement from Comunidad de Madrid Blood Centre and used to isolate human T cells. Peripheral blood mononuclear cells (PBMCs) were obtained by density gradient centrifugation (Lymphoprep^{™}, StemCell Technologies), and T cells were then isolated by depletion of monocytes, neutrophils, eosinophils, B cells, dendritic cells, NK cells, granulocytes and erythroid cells using a cocktail of PE-conjugated antibodies against linage-specific markers (CD11b, CD13, CD14, CD19, CD25, CD33, CD56, CD123), together with a mouse anti-human CD235a (Glycophorin A) purified antibody labelled with an anti-mouse-PE secondary antibody **(Table 1).** All stainings were performed in the dark at 4 °C. Subsequently, cells were incubated with anti-PE magnetic beads (Miltenyi Biotec) following manufacturer's instructions. Isolation of highly-purified unlabelled T cells was achieved by depletion of magnetically labelled cells using negative selection columns (MACS LD Columns, Miltentyi Biotec).

### 3. Flow cytometry analysis

All flow cytometry stainings were performed in the dark at 4 °C. Surface marker expression was determined by staining of 1-2 x 10⁵ cells in ice-cold flow cytometry buffer (PBS 1X, 1% BSA, 1% FBS and 0.01% sodium azide). Fluorochrome- or biotin-labelled primary mAbs and secondary antibodies used for flow cytometry staining are specified in **Table 2.** Isotype-matched irrelevant antibodies were used as negative controls to define background fluorescence. For *ex vivo* samples, FcRy receptors were first blocked with rat anti-mouse CD16/CD32 antibodies **(Table 2)** for 15 min at 4 °C. For intracellular stainings, cells were previously fixed and permeabilized using CytoFix/CytoPerm (BD Biosciences). One-color flow cytometry acquisition was performed using a FACSCalibur cytometer and CellQuest software (BD Biosciences), and up to seven-colours flow cytometery acquisition was performed using FACSCanto II cytometer and FACSDiva software (BD Biosciences). Flow cytometry data analysis was performed with FlowJo X (LLC software).

### 4. CAR-expressing lentiviral production and T-cell transduction, activation and expansion

Lentiviral particles were generated by co-transfection of HEK-293T-packaging cells using jetPEI reagent (Polyplus) and the following plasmids: the LV construct (either pCCL-GFP-Mock, pCCL-CARpTα.28 or pCCL-CARpTα.41BB), the pMD2.G plasmid (encoding the VSV-G envelope), the pMDLg/pRRE (encoding the gag/pol proteins) and the pRSV-Rev (encoding Rev protein), following manufacturer's instructions, and concentrated by ultracentrifugation.

Jurkat T cells (10⁵ cells) were lentivirally transduced by spin centrifugation (1 h at 1,800 rpm) using 1 ml of LV supernatants (containing either GFP-Mock, CARpTα.41BB or CARpTα.28), in the presence of 8 µg/ml hexadimethrine bromide (Polybrene, Sigma Aldrich) in 24-well plates. After 24 h at 37 °C, viral particles were washed-off and cells were resuspended in fresh RPMI 10% FBS. Transduction efficiency was checked 48 h later by detection of GFP by flow cytometry and CARpTa surface expression was detected using a Biotin-coupled anti-F(ab')₂ goat anti-mouse IgG-F(ab')₂ secondary Ab and Streptavidin-APC **(Table 2).**

Human peripheral T cells were plated (10⁶ cells/ml) in RPMI medium 10% fetal bovine serum (FBS), 2 mM glutamine, 1% penicillin/streptomycin, 10 mM Hepes, and stimulated with Dynabeads Human T-Activator CD3/CD28 (ThermoFisher) at a 3:1 bead:cell ratio at 37 °C. After 24 h, CD69 expression was analysed by flow cytometry to determine efficient cell activation and was compared with non-stimulated T cells. Then, activated T cells were transduced with LV supernatants (SNs) in the presence of recombinant human IL-7 (200 IU/ml) (National Institute for Biological Standards and Control, NIBSC) and IL-15 (20 IU/ml) (PreproTech) at a multiplicity of infection (MOI) of 10. After 2 days, virus SN was diluted with fresh medium supplemented with IL-17/15. Cells were expanded at 37°C for a maximum of 10-14 days in RPMI 10% plus IL-7/15. Beads were removed from culture on day 5 post-stimulation, following manufacturer's instructions. Transduction efficiency was determined by flow cytometry analysis of GFP expression, and CARpTα.41 BB surface expression was detected by flow cytometry as described above.

### 5. In vitro functional assays of CARpTα-armed T cells

### 5.1. Analysis of CARpTα-mediated T-cell activation in Jurkat cells

To analyse T-cell activation induced by the CARpTa constructs upon recognition of the pre-TCR complex, CARpTa-transduced (either CARpTα.28- or CARpTα.41BB-transduced) or non-transduced (NT) Jurkat cells, used as negative control, were cultured in the presence of either pre-TCR+ (SupT1) or pre-TCR- (REH) target cells in 48-well plates at a final concentration of 1.2 x 10⁶ cells/ml in RPMI 10% FBS for 16 h at 37 °C. Different effector: target (E:T) cell ratios were set up in culture, using the following E:T cell numbers: 1:0 E:T ratio, 600,000 effector cells cultured in the absence of target cells to determine the basal activation status of the effector cells; 1:1 E:T ratio, 300,000 effector and 300,000 target cells; 1:5 E:T ratio, 100,000 effector and 500,000 target cells. After 16 h of co-culture, expression of CD69 early activation marker in CARpTa or NT Jurkat cells was analysed by flow cytometry using an anti-CD69-PE mAb **(Table 2).** CD3-APC, CD4-PeCy5 and CD8-PeCy7 or CD19-PeCy7 antibodies were used to discriminate between effector Jurkat cells and target cells **(Table 2),** and surface CARpTa expression was detected as indicated in Section 5.1.

### 5.2 Antibody blocking assays

To perform antibody blocking assays, pre-TCR+ SupT1 target cells were pre-incubated with 5 µg/ml of either anti-human pTa mAb or isotype-matched control mAb (mouse lgG2a) for 30 min at 4 °C. Then, unbound antibody was washed out and 100,000 CARpTα.41BB-transduced Jurkat cells were co-cultured with 500,000 target cells (1:5 E:T ratio) in RPMI 10% supplemented with either 5 µg/ml anti-human pTa mAb or control mAb. To assess the basal activation status of the effector cells, 600,000 CARpTα.41BB-armed Jurkat cells were cultured in the absence of target cells (1:0 E:T ratio) in RPMI 10% supplemented with either anti-human pTa mAb or control mAb. Expression of CD69 activation marker was analysed after 16 h by flow cytometry using CD69-PE mAb together with CD4-PeCy5, CD8-PeCy7 and CD3-APC mAbs **(Table 2).**

### 5.3. Analysis of CARpTα-induced cytotoxic potential and T-cell activation of primary human T cells

For cytotoxic assays, pre-TCR+ (SupT1 and JR-Mig pTα) or pre-TCR- (REH, JR-TCRα, JR-Mig R1, K562 and 721-22) target cells were previously labelled with CellTrace^{™} Violet (ThermoFisher) following manufacturer's instructions. Then, 20,000 CellTrace^{™} Violet-labelled target cells were plated in U-shape 96-well microplates in RPMI 10% FBS, and either CARpTα.41BB-transduced or GFP-Mock-transduced effector primary human T cells were added at the indicated E:T cell ratios (i.e. for a 2:1 E:T ratio, 40,000 effector cells were co-cultured with 20,000 target cells and for a 0.5:1 E:T ratio, 10,000 effector cells were co-cultured with 20,000 target cells). Constitutive activation of primary human T cells transduced with either CARpTα.41BB or GFP-Mock vectors was determined in cultures set up at the cell concentrations indicated for each E:T ratio but in the absence of effector cells. As control, target cells were cultured alone to determine spontaneous cell death. Induction of CD69 activation marker in effector T cells (CellTrace^{™} Violet-negative) was assessed 16 h later by flow cytometry as readout of T-cell activation using CD69-PE mAb **(Table 2)**. Cytotoxic activity was determined by flow cytometry by analysing the percentage of alive target cells (7-AAD-negative, CellTrace^{™} Violet-positive), and quantification of absolute cell counts of alive target cells was determined using AccuCheck Counting Beads (ThermoFisher).

### 5.4. Determination of pro-inflammatory cytokines release

To assess the production of pro-inflammatory cytokines by CARpTα.41 BB-T cells upon pre-TCR recognition, 20,000 CARpTα.41BB- or GFP-Mock-transduced primary human T cells were co-cultured with 20,000 pre-TCR+ (SupT1 and JR-Mig pTα) or 20,000 pre-TCR- (K562) target cells (1:1 E:T cell ratio) in U-shape 96-well microplates at 37 °C. Culture supernatants were collected 16 h later and production of the pro-inflammatory cytokines IL-2, tumour necrosis factor α (TNFα), and interferon γ (IFN-γ) was determined by an enzyme-linked immunosorbent assay (ELISA) using human IL-2, TNFα, IFN-γ DuoSet ELISA and DuoSet ELISA Ancillary Reagent Kit 2 (R&D Systems), following manufacturer's instructions.

### 6. Generation and functional validation of anti-pTα Antibody-Drug Conjugates (ADCs)

### 6.1. Generation of anti-pTα ADCs

The purified anti-pTα was conjugated to the cytotoxic agent Mertansine Toxin (DM1) via a non-cleavable MCC attachment group or to Monomethyl auristatin E toxin (MMAE) via valine-citruline protease-cleavable linker. Custom Antibody Services (CABS) from Institute for Advanced Chemistry of Catalonia was responsible for the development, production and purification of ADCs.

### 6.2. Pre-TCR labeling and internalization with anti-pTα-ADCs

SupT1 cells were pulsed with 20 µg/ml of anti-pTα mAb or anti-pTα ADCs (pTα-DM1 or pTα-MMAE) at 4°C for 30 min, then washed with ice-cold PBS to remove unbound antibody and chased for the indicated times at 37°C. Then, cells were washed with ice-cold PBS and the primary antibody was detected using FITC-conjugated goat anti-mouse (H+L) antibody (Invitrogen).

### 6.3. Cell proliferation and cell cycle analysis

Cells were seeded (2x10⁵ cells/ml) in 48-well plates in the presence of 5 µg/ml anti-pTα mAb, anti-pTα ADCs or isotype-matched irrelevant controls for 4 days at 37°C, 5% CO₂. Control samples were treated with medium alone. Thereafter, cells were collected, washed and counted. For cell cycle analysis, 4x10⁵ cells were incubated with 5 µg/ml of Hoechst 33342 (Sigma) at 37°C for 45 min, washed with ice-cold PBS, and analyzed by flow cytometry using a FACSCanto II and FACSDiva software. Flow cytometry data analysis was performed using FlowJo vX (LLC software) (BD Biosciences).

### 7. In vivo mouse models

Mice were kept under specific pathogen-free (SPF) conditions and used in accordance with the guidelines approved by the Animal Experimentation Ethics Committee of the Spanish National Research Council. Experiments were performed in a biosafety level 2 room (BSL-2) at the Animal Facility at Centro de Biologia Molecular Severo Ochoa (CBMSO, CSIC-UAM).

### 7.1. In vivo SupT1-Luc preclinical mouse model

Sub-lethally irradiated (1.5 Gy) 6-10-week-old NOD.Cg-*Prkdc*^{scid}γc^{-/-} (NSG; The Jackson Laboratory) immunodeficient mice were subjected to intravenous (i.v.) injection with the pre-TCR+ SupT1 T-ALL cell line previously transduced with a lentiviral construct encoding luciferase and GFP reporter genes separated by an IRES (pHR-SIN-Luc-IRES-emGFP) (Cuesta-Mateos, et al. 2020 Biomarker Research, 8:54 (1 x 10⁶ cells per mice). A single dose of CARpTα.41 BB or GFP-Mock primary human T cells was i.v. administered (5 x 10⁶ cells per mice) two days after leukaemia infusion. Mice were monitored visually on a daily basis for their general appearance, and weekly for tumour growth by non-invasive whole-body bioluminescence imaging (IVIS). Mice that showed clear signs of distress (cachexia) were sacrificed following humane endpoints guidelines. BM, spleen and PB were isolated from all sacrificed mice and processed for flow cytometry analysis, as described in section 7.3.

### 7.1.1. In vivo bioluminescence analysis

For bioluminescence assessment of tumour burden, mice were firstly anesthetised using Isoflutek^{®} (Laboratorios Karizoo, S.A) and subsequently intraperitoneally injected with 150 mg/kg of D-Luciferin (PerkinElmer) and monitored using the Xenogen IVIS Lumina II imaging system (Caliper Life Sciences). Photon flux emitted by luciferase-expressing cells was measured for 10 min as average radiance (photons/sec/cm²/sr). Imaging analysis was performed using the Living ImageTM software 3.2 (Caliper Life Sciences).

### 7.2. In vivo T-ALL patient-derived xenograft (PDX) preclinical model

Sub-lethally irradiated (1.5 Gy) 6-10-week-old NSG immunodeficient mice were i.v. injected with primary pre-TCR+ T-ALL blasts from patient samples (T-ALL 3: 0.5-1 x10⁶cells per mice or T-ALL 42: 2x10⁶ cell per mice). Leukemia engraftment in PB was monitored weekly by flow cytometry using anti-human CD45-APC, CD7-PE and CD5-FITC antibodies. Animals were subjected to the corresponding therapy when PB leukemia engraftment was >1%.

### 7.2.1. In vivo CARpTα-T cell therapy

A single dose (2 x 10⁶ cells per mice) of primary human CARpTα.41BB-armed or GFP-Mock-transduced T cells was i.v. injected in mice subjected to leukemia infusion at least two days before. Mice were then monitored visually on a daily basis for their general aspect and weekly for leukaemia expansion in PB by bleeding. Mice were sacrificed at week 11 post-leukemia infusion when signs of graft versus host disease (GvHD) were evident. Analysis of weekly PB samples, and BM and spleen at sacrifice was done by flow cytometry.

### 7.2.2. In vivo anti-pTα ADC therapy

Mice injected with T-ALL primary samples were homogeneously divided into different groups that were treated with either vehicle (PBS), anti-pTα-DM1 (3 and 10 mg/Kg, twice per week) or VxL, as the standard patient treatment, consisting of Vincristine (0.15 mg/kg) once weekly for 3 weeks, dexamethasome (5 mg/Kg) and and L-asparaginase (1000 U/Kg) daily during 5 days for 3 weeks. In a second experiment mice were treated with either anti-pTα-MMAE (3 and 6 mg/Kg) twice per week or anti-IgG2a-MMAE as control.

### 7.3 Organ collection and processing for ex vivo analysis

Blood samples were obtained from the submandibular vein and collected in heparin-containing tubes (Chiesi España, S.A.). Then, 1 ml of red blood cell (RBC) lysis buffer A (0.15 M NH₄Cl; 10 mM KHCOs; 0.1 mM EDTA) was added to each sample and incubated for 5 min at room temperature. Samples were then centrifuged for 10 min at 7,000 rpm at 4 °C. Cell pellets were washed twice in flow cytometry buffer and stained for flow cytometry analysis with specific antibodies (Table 2).

Cells from spleens were isolated by mechanical disruption and filtration through a 40 µm cell strainer (Falcon^{®}). BM cells were obtained by perfusion with PBS 1X of both femora and tibiae from mouse legs. Samples suspended in RBC lysis buffer B (9 vol. NH₄Cl 0.83%; 1 vol. Tris-HCl 2.6% pH7.6) were incubated for 5 min at room temperature. Then, lysis reaction was neutralized by addition of RPMI 2% FBS, samples were filtered through 70 µm filters (Filcon, BD Biosciences) and subjected to centrifugation. Finally, cell pellets were washed twice in flow cytometry buffer and stained for flow cytometry analysis with specific antibodies **(Table 2).**

### 9. Statistical Analysis

Statistical analyses were performed using Prim 8.0.1 software (GraphPad, La Jolla, CA, USA). Statistical significance was determined by unpaired 2-tailed Student's *t*-test when comparing two groups or one-way ANOVA when comparing more than two groups. To account for multiple comparisons, data were corrected using False Discovery Rate (FDR) method. Kaplan-Meier survival curves were compared using the log-rank test. In all cases, significance level was set at alpha = 0.05.

### RESULTS

### 1. In vitro validation of engineered anti-pTα scFv

In order to evaluate the specificity of the engineered anti-pTα scFv isolated as described above, we assayed by flow cytometry the reactivity of SupT1 T-ALL cells with the purified anti-pTα scFv, in comparison with the reactivity of equivalent amounts of an anti-pTα mAb **(****Figure 1****).** We found that the anti-pTα scFv products were able to specifically recognise the pre-TCR expressed at the cell surface of SupT1 cells, although at lower levels than the complete anti-pTα mAb. Importantly, none of the purified anti-pTα scFv samples were reactive with the TCRαβ+ Jurkat cell line used as negative control, this confirming specificity of the engineered anti-pTα scFv.

### 2. Engineering and validation of two distinct 2nd generation CARpTα constructs and in vitro comparison.

### 2.1. Generation of CARpTα constructs

FDA-approved CAR-T therapies are currently based on second-generation CAR constructs that include either CD28 or 4-1BB as co-stimulatory domains. Previous studies reported that CD28-based CARs usually induce a robust proliferative response and an effector memory phenotype of T cells, whereas 4-1BB-based CARs elicit a more progressive response, presenting enhanced persistence and central memory differentiation (Kawalekar et al. 2016 Immunity. 16;44(2):380-90, doi: 10.1016/j.immuni.2016.01.021.). However, the therapeutic advantage of one or the other seemed dependent on additional CAR structural cues, including the nature of the hinge and TM regions, and the scFv itself (Mazinani & Rahbarizadeh, 2022. Review Biomark Res. 10(1): 70).

In this invention, we addressed the generation of two different 2^{nd}-generation CAR constructs against the pTα chain of the pre-TCR that incorporated either CD28 or 4-1BB as co-stimulatory domains, and differ as well in the hinge and transmembrane regions **(****Figure 2****),** also performing a parallel *in vitro* comparison of both products. The two CARpTa were named CARpTα.41BB and CARpTα.28, depending on the co-stimulatory domain used (4-1BB and CD28, respectively). Complete CARpTa structures are detailed in **Table 3.**

After validating the specificity of the anti-pTα scFv, it was cloned into two different second-generation CAR backbones (CAR.41 BB and CAR.28) **(****Figure 2****)** by means of PCR amplification, enzyme digestion and Gibson assembly. After validating correct insertion and sequence of the CARpTa constructs generated, both CARpTα.41 BB and CARpTα.28 were cloned into the pCCI lentiviral vector. Lentiviral particles containing either each CARpTa LV vector or the pCCL-GFP-Mock LV vector, used as a negative control, were produced using HEK-293T cells. LV particles were then used to transduce the Jurkat cell line and efficiency of transduction was evaluated by detection of GFP expression by flow cytometry at 48 h post-transduction. Results showed that Jurkat cells were efficiently transduced in all the cases, although better transduction efficiency was observed for CARpTα.41 BB LV particles (95.1%) compared to CARpTα.28 LV particles (78.2%) **(****Figure 3A****)**

### 2.2. In vitro functional comparison of CARpTα.41 BB vs. CARpTα.28

Analysis of CARpTa expression at cell surface of Jurkat cells over time revealed huge differences between both CARpTα.41 BB and CARpTα.28 constructs **(****Figure 3B****).** Whereas CARpTα.41 BB was expressed and maintained over time at high levels at the cell surface of Jurkat cells, surface expression of CARpTα.28 was significantly reduced overtime, being minimally expressed by around 10% of the cells at day 21 post-transduction. Notably, loss the CARpTα.28 correlated with a parallel loss of total GFP+ Jurkat cells (transduced cells) over time **(****Figure 3B****).** These data, suggest that CARpTα.28-transduced, but not CARpTα.41 BB-transduced Jurkat cells are lost over time in culture.

We next compared the functional potential of the two engineered CARpTa constructs to specifically recognize the pTa component of the human pre-TCR expressed on the surface of T-ALL cells, and to induce cellular activation upon pre-TCR recognition. To this end, CARpTα.28- or CARpTα.41 BB-expressing Jurkat cells were co-cultured with the human pre-TCR+ SupT1 T-ALL cell line at different E:T cell ratios. Cell activation was analysed after 16h of co-culture by assessing induction of the CD69 early activation marker in transduced Jurkat cells, which were gated by flow cytometry based on CD4 and CD8 expression levels, together with GFP and surface CARpTα expression **(****Figure 4A****).** Our results showed that, upon co-culture with pre-TCR+ SupT1 cells, both CARpTα.28- and CARpTα.41 BB-expressing Jurkat cells, became activated and upregulated CD69 expression in a E:T-dependent manner **(****Figure 4B****).** However, CD69 mean fluorescence intensity analysis also revealed a significant increase of CD69 basal expression levels on CARpTα.28-transduced Jurkat cells compared to non-transduced control cells in the absence of pre-TCR interaction **(****Figure 4C****),** indicating that CARpTα.28 expression per se induces tonic signalling and results in constitutive activation of Jurkat cells. In contrast, no significant cell activation was induced in CARpTα.41 BB-transduced Jurkat cells, which showed basal CD69 expression levels similar to those observed in non-transduced Jurkat cells **(****Figure 4C****).** Therefore, our data suggest that CARpTα.28 expression selectively results in tonic signalling induction in the absence of the target, this leading to a non-specific activation of CAR-expressing T cells, which could result in exhaustion and activation-induced cell death. Importantly, this non-specific effect was not observed in CARpTα.41 BB-transduced Jurkat cells.

Given the results obtained for the CARpTα.28 construct regarding loss of transduced cells and high tonic signalling, we decided to focus on the CARpTα.41 BB construct for further generation of CARpTa-bearing T cells.

### 2.3. In vitro functional validation of CARpTα.41 BB construct.

We functionally validated the specificity of the CARpTα.41 BB by assessing its capacity to induce the activation of CARpTα.41BB-armed Jurkat cells cultured with either a pre-TCR+ (SupT1) or a pre-TCR- (REH) cell line used as negative control **(****Figure 5A****).** Results showed that CARpTα.41 BB-transduced Jurkat cells significantly upregulated CD69 expression levels, becoming activated, after incubation with the pre-TCR+ SupT1 cell line in an E:T-dependent manner. In contrast, no CD69 upregulation was observed after co-culture of CARpTα.41 BB-armed Jurkat cells with the pre-TCR- REH cell line **(****Figure 5B-5C****).** Therefore, recognition of surface pre-TCR by CARpTα.41 BB is specific and functionally efficient, resulting in T-cell activation. Confirming that CD69 expression was specifically induced by CARpTα.41 BB signalling, we found that non-transduced Jurkat cells were unable to upregulate CD69 upon interaction with either SupT1 or REH cells **(****Figure 5C****).**

Formal proof that CARpTα.41 BB-mediated T cell activation was specifically induced upon interaction with the pre-TCR was further provided by antibody blocking assays. CARpTα.41BB-armed Jurkat cells were co-cultured with pre-TCR+ SupT1 target cells, but the CARpTα.41BB/pre-TCR interaction was prevented via pre-incubation of target cells with an anti-pTα mAb (**Figure 6A**). Our results showed that pre-incubation of target cells with the anti-pTα mAb, but not with an isotype-matched irrelevant mAb, impaired the activation of CARpTα.41 BB-armed Jurkat cells, as assessed by analysis of CD69 induction **(****Figure 6B-6C****)**. Therefore, we concluded that T-cell activation mediated by CARpTα.41BB was specifically induced after binding to the pre-TCR complex.

### 3. Generation and in vitro functional validation of CARpTα.41 BB-armed primary human T cells

### 3.1. Generation and expansion of CARpTα.41BB-armed primary human T cells

After validating the specificity and function of the CARpTα.41BB construct in Jurkat cells, we engineered primary human T cells armed with the CARpTα.41BB. To this end, primary human T cells were isolated by negative selection from PBMCs suspensions obtained from healthy donor blood samples via Ficoll-Hypaque density gradient **(****Figure 7A****)** T-cell isolation was performed by negative column selection, rendering a cell suspension enriched up to 90% (89.63 ± 5.95%, mean ± SD. N = 4 different donors) in CD3+ either CD4+ or CD8+ T cells (Figure 7A-B). Highly purified T cells were then activated before transduction using anti-CD3/CD28 beads, which mimic T-cell stimulation via TCRαβ/CD28 and induced CD69 expression **(****Figure 7B****)**. Activated T cells were transduced with either CARpTα.41BB or GFP-Mock lentiviral particles and expanded in the presence of IL-7 and IL-15. Transduction efficiency was analysed by flow cytometry using GFP as a reporter gene and CARpTa surface expression was directly assessed by flow cytometry using an anti-mouse F(ab')₂ antibody **(****Figure 7D****).** Results showed high transduction efficienc of primary human T cells with the CARpTα.41BB (up to 63% GFP+ cells). Importantly, all transduced cells displayed the CARpTα.41BB at the cell surface, and both CD4+ and CD8+ cell subsets were equally transduced **(****Figure 7D****).**

### 3.2. Specific activation of CARpTα.41BB-armed primary human T cells upon pre-TCR recognition

Transduced CARpTα.41BB primary human T cells were assayed *in vitro* for their capacity to become specifically activated by the pre-TCR-expressing SupT1 cell line in comparison with the pre-TCR-negative REH cells **(****Figure 8****).** Results demonstrated that, in contrast to GFP-Mock-transduced T cells, CARpTα.41BB T cells significantly upregulated their CD69 expression levels, indicating that they become activated after incubation with pre-TCR+ SupT1 cells in an E:T-dependent manner **(****Figure 8A****).** In contrast, no significant CD69 induction was observed after co-culturing CARpTα.41BB-armed T cells with the pre-TCR- REH cell lines. (Figure **8A-8B****).** Confirming specific activation of CARpTα.41BB-armed T cells upon pre-TCR recognition, similar results were observed when using the pre-TCR+ JR-Mig pTa cell line as a target. in comparison with the pre-TCR- JR-Mig R1 and JR-TCRα cell lines used as negative control **(****Figure 9A-9B****).**

### 3.3. CARpTα.41BB-armed primary human T cells display high in vitro cytotoxic potential against pre-TCR+ T-ALL cell lines

To evaluate the anti-leukemic potential of CARpTα.41BB T cells, we next assessed their killing activity *in vitro* and their capacity to secrete pro-inflammatory cytokines against pre-TCR+ T-ALL cell lines (SupT1 and JR-Mig-pTα) in comparison with pre-TCR- cell lines (K562 and 721-22), used as negative controls (Figure 10A). Compared to GFP-Mock T cells, CARpTα.41BB T cells showed robust cytotoxic activity against pre-TCR+ T-ALL cell lines, while no significant cytolysis of pre-TCR-cells was observed **(****Figure 10A****).** Cytotoxic potential of CARpTα.41BB-armed T cells was found dependent on the E:T cell ratio. Remarkably, up to 80% and 90% of specific lysis of JR-Mig-pTα cells was observed at E:T cell ratios as low as 0.25:1 and 0.5:1, respectively, while pre-TCR- control cell lines were not significantly affected even at the highest E:T cell ratios.

We also evaluated the cytokine secretion potential of CARpTα.41BB-armed T cells *in vitro* against pre-TCR+ target cells (SupT1 and JR-pTα) or pre-TCR- target cells (K562 and 721-22). Results confirmed that, in contrast to GFP-Mock-transduced T cells, pre-TCR-mediated activation of CARpTα.41BB-armed T cells co-cultured with pre-TCR+ cell lines became activated and produced high levels of the pro-inflammatory cytokines IL-2, TNFα and IFNγ on co-culture with pre-TCR+ cell lines, while no cytokine production was observed upon co-culture with pre-TCR- cells **(****Figure 10B****).** These results support that specific recognition of the pTα chain of the human pre-TCR by the CARpTα.41BB triggers robust *in vitro* cytotoxic activity in CARpTα.41BB-armed primary human T cells.

### 4. In vivo therapeutic potential of CARpTα.41BB-armed T cells

Our next aim was to validate the efficacy of the anti-pre-TCR CAR-T cell-based immunotherapy *in vivo,* with the aim of providing proof-of-principle of its suitability for pre-TCR+ T-ALL patient treatment. To this end, we took advantage of two preclinical models of immunodeficient mice transplanted either with Luciferase (Luc)-expressing SupT1 cells or primary patient-derived T-ALL xenografts (PDX).

### 4.1. Therapeutic efficacy of CARpTα-armed T cells in a preclinical model of mice transplanted with Luc-expressing SupT1 cells.

For *in vivo* validation of the proposed CARpTa-T cell therapeutic strategy, we first used sub-lethally irradiated NSG mice that were transplanted by intravenous (i.v.) injection with 1 x 10⁶ Luc-expressing SupT1 cells 2 days before i.v. infusion of either 5 x 10⁶ GFP-Mock-transduced or 5 x 10⁶ CARpTα.41BB-armed T cells **(****Figure 11A****).** Leukemia progression was followed up weekly by bioluminescence imaging and by flow cytometry analysis of BM and spleen samples at the end of the experiment. Results showed that administration of CARpTα.41BB-armed T cells significantly hampered T-ALL progression and increased overall mouse survival, as compared to treatment with GFP-Mock-transduced T cells **(**Figure 11B-D). Moreover, flow cytometry analysis of SupT1 leukaemic cells infiltrating the BM revealed significant differences in CD3 expression levels between mice treated with either CARpTα.41BB-armed T cells or transduced with GFP-Mock **(**Figure 11E-F). In fact, SupT1 cells recovered from the GFP-Mock control group displayed surface CD3 expression levels equivalent to those recovered from untreated animals. In contrast, SupT1 leukaemic cells surviving in mice treated with CARpTα.41BB-armed T cells showed very low-to-undetectable CD3 surface expression levels, this indicating that *in vivo* CARpTα.41BB-T cell immunotherapy selectively eliminates pre-TCR+ leukemic SupT1 cells.

### 4.2. Potent anti-leukemic activity of CARpTα-armed T cells in a T-ALL patient-derived xenograft (PDX) in vivo model

To further validate the *in vivo* efficacy of the CARpTα.41BB-T cell immunotherapy, we used a more relevant preclinical T-ALL PDX model **(****Figure 12A****)**, in which sub-lethally irradiated NSG mice were i.v. injected with 1 x 10⁶ primary T-ALL blasts from a patient sample, and two days later they were infused with 2 x 10⁶ GFP-Mock- or CARpTα.41BB-transduced human primary T cells. Leukemia engraftment was then followed up weekly by bleeding BM and spleen samples were analysed at the end of the experiment, at week 11, when all animals were euthanized owing to the appearance of greft versus host disease (GvHD) symptoms. Mice were considered positive for leukemia engraftment when they showed > 0.4% of T-ALL blasts in the PB or > 2.0% in BM or spleen. Kinetic studies showed that engraftment of T-ALL cells gradually increased over time in the PB of GFP-Mock-T cells-treated mice **(****Figure 12B****),** with 5 out of 5 (100%) of those mice showing a huge leukemia burden in BM and spleen at sacrifice **(****Figure 12B****).** In contrast, 4 of 5 (80%) of mice given CARpTα.41BB-T cell therapy were leukemia- free even after 11-weeks of CARpTα.41BB-Tcell infusion (**Figure 12B**), providing proof-of-concept of the efficacy of our CARpTa-based immunotherapy for T-ALL.

### 5. Resutlados del Ensayo Anticuerpo-Toxina (ADC)

He insertado las figuras correspondientes a este ensayo como Figuras 14 a 17.

### 5. In vitro validation of pTα-specific antibody-drug conjugates (ADCs)

To generate pTα-specific antibody-drug conjugates (ADCs), lysine-specific drug conjugation of the anti-pTα mAb was carried out by the Custom Antibody Services (CABS) from Institute of Advanced Chemistry of Catalonia, using two different cytotoxic agents Mertansine Toxin (DM1) or Monomethyl auristatin E toxin (MMAE). To determine whether the pTα-ADCs retained their binding capacity toward human pre-TCR, we tested the potential of these ADCs to interact with either pre-TCR⁺ (SupT1, JR-Mig-pTα) or pre-TCR- (Jurkat, JR-Mig-R1) cell lines by flow cytometry **(****Figure 13****)**. As expected, anti-pTα mAb was unable to bind to the surface pre-TCR negative Jurkat or JRMigR1 cell lines, neither anti-pTα-ADCs showed reactivity against these cells. However, both ADCs as well as the conventional anti-pTα mAb recognized a CD3-associated complex expressed at the surface of cell lines known to express the pre-TCR **(****Figure 13A****),** indicating that they bind to the pTα chain of the pCD3-associated pre-TCR.

### 5.1. pTα-specific ADCs bind and rapidly internalize the human pre-TCR expressed at the cell surface.

To investigate whether the anti-pTα ADCs may display appropriate characteristics for cargo intracellular delivery, we next analysed the internalization potential of the pre-TCR complex bound to the anti-pTα ADCs reagents, using a flow cytometry internalization assay. We observed that percentages of cells expressing surface pre-TCR decreased significantly (> 60-70%) upon short-time incubation with the anti-pTα ADCs, indicating surface internalization of the pre-TCR, and similar internalization kinetics were observed with the uncoupled anti-pTα mAb **(****Figure13B****).**

### 5.2. Anti-pTα ADCs specifically impair cell cycle progression and in vitro cell proliferation of pre-TCR-expressing T-ALL cells.

We next investigated whether the observed internalization of the anti-pTα ADCs could have an impact on the proliferation of pre-TCR-expressing cells. To this end, we assessed cell cycle status and cell numbers of pre-TCR+ leukemic cells treated with the anti-pTα ADCs or with the conventional unconjugated anti-pTα mAb, as compared with pre-TCR- cells. We found a selective G2/M cell cycle arrest of pre-TCR+ leukemic cells (SupT1) incubated with the anti-pTα ADCs, attributed to the mitotic blockage effect of the DM1 and MMAE cytotoxic agents, as this effect was not observed in the presence of unconjugated anti-pTα mAb **(****Figure 14A****).** Importantly, blockade at the G2/M cell cycle phase was specific of pre-TCR-expressing leukemic cells, and was observed with both DM1- **(****Figure 14B****)** and MMAE-conjugated **(****Figure 14D****)** anti-pTα ADCs, while no impact on the cell cycle was observed by isotype-matched irrelevant drug-conjugated controls. As expected, cell cycle arrest resulted in a significant and specific inhibition of cell proliferation of pre-TCR+ leukemias **(****Figure 14C-14E****),** which provides evidence of a strong *in vitro* therapeutic impact of anti-pTα ADCs on pre-TCR+ T-ALL leukemias.

### 5.3. In vivo therapeutic impact of anti-pTα ADCs on pre-TCR-expressing T-ALL

To analyze the *in vivo* efficacy of a potential immunotherapy for pre-TCR+ T-ALL, based on anti-pTα ADC administration, sublethally irradiated NSG mice (1.5 Gy) were i.v. injected with primary patient-derived T-ALL xenografts previously expanded in NSG mice. Animals were examined at different days post-transplantation for the presence of human leukemic cells in peripheral blood (PB) and the disease was considered established when animals showed >1% leukemic cells in the PB (>8 weeks post-transplantation). At that point, treatment was initiated by intraperitoneal administration of the anti-pTα-DM1 (3 or 10 mg/Kg), VxL, representing conventional chemotherapy administered to patients or vehicle (PBS) as control, twice a week for 3 weeks **(****Figure 15A****).** A selective impairment of leukemic progression was observed in the PB of animals treated with the standard VxL and with anti-pTα-DM1, as compared with vehicle-treated animals **(****Figure 15B****).** However, phenotypic analysis demonstrated a selective loss of pre-TCR-bearing leukemic cells in animals treated with anti-pTα-DM1, as compared with a general cellular loss observed in VxL-treated mice, this supporting the pre-TCR specificity of the anti-pTα-DM1 reagent (**Figure 15C**). Notably, although treatment cessation led to leukemia regrowth (**Figure 15B**), survival of treated mice was significantly improved compared to that of control animals **(****Figure 15D****).** Moreover, identical results were obtained using the anti-pTα-MMAE ADC for treatment of a different T-ALL patient xenograft **(****Figure 16****).** Therefore, these results provide formal proof of the efficacy of a novel anti-pTα ADC-based therapy against pre-TCR+ T-ALL.

### Tables of the Examples:

**Table 1. Antibodies used for isolation of human peripheral blood T cells**

| **Specificity** | **Conjugat e** | **Isotype** | **Clone** | **Company** | **Cat. No** |
|---|---|---|---|---|---|
| CD11b | PE | IgG1 Mouse | ICRF44 | BD Biosciences | 555388 |
| CD13 | PE | IgG1 Mouse | WM15 | BD Biosciences | 555394 |
| CD14 | PE | IgG2b Mouse | MΦP9 | BD Biosciences | 345785 |
| CD19 | PE | IgG1 Mouse | HIB19 | BD Biosciences | 555413 |
| CD25 | PE | IgG1 Mouse | MA251 | BD Biosciences | 555432 |
| CD33 | PE | IgG1 Mouse | P67.6 | BD Biosciences | 345799 |
| CD56 | PE | IgG2a Mouse | MEM-188 | Thermo Fisher | MHCD56 04 |
| CD123 | PE | IgG1 Mouse | 9F5 | BD | 555644 |
| CD235a | - | Ascites | 10F7 | - | - |
| Anti-Mouse IgG (H+L) | PE | IgG Goat | - | Southern Biotech | 1032-09 |

**Table 2. Antibodies against human antigens used in flow cytometry assays.**

| Specificity | **Conjugate** | **Clone** | **Company** |
|---|---|---|---|
| CD3 | PE | UCHT1 | BD Biosciences |
| | APC | UCHT1 | BD Biosciences |
| CD4 | PE | SFCI12T4D11 | Beckman Coulter |
| | PE-Cy5 | 13B8.2 | Beckman Coulter |
| CD8 | PE-Cy7 | RPA-T8 | BD Biosciences |
| CD19 | PE | HIB19 | BD Biosciences |
| | PE-Cy7 | SJ25C1 | BD Biosciences |
| CD25 | PE | M-A251 | BD Biosciences |
| | APC | BC96 | eBioscience |
| CD45 | V450 | 2D1 | BD Biosciences |
| CD45RA | PE | MEM-56 | Thermo Fisher |
| CD45RO | PE | UCHL1 | Thermo Fisher |
| CD52 | PerCP/Cy5.5 | HI186 | Biolegend |
| CD56 | PE | MEM-188 | Thermo Fisher |
| | PE-Cy5 | N901 (NKH-1) | Beckman Coulter |
| CD62L | PE | FN50 | BD Biosciences |
| | APC | CH/4 | Thermo Fisher |
| CD69 | PE | FN50 | BD Biosciences |
| | APC | CH/4 | Thermo Fisher |
| CD223 (LAG-3) | PE | T47-530 | BD Biosciences |
| CD279 (PD-1) | APC | EH12.2H7 | Biolegend |
| CD366 (TIM-3) | PE | 7D3 | BD Biosciences |
| Histidine tag | Purified | AD1.1.10 | Bio-Rad |
| TCR α/β | PE-Cy5 | IP26 | Beckman Coulter |
| Streptavidin | APC | --- | Biolegend |
| anti-Mouse IgG, F(ab')₂ Fragment Specific | Biotin | --- | Jackson Immunoresearch |
| anti-mouse CD16/CD32 | Purified | 2.4G2 | BD Biosciences |
| anti-mouse Ig Kappa light chain | PE | 187.1 | BD Biosciences |
| anti-mouse Ig Lambda light chain | FITC | R26-46 | BD Biosciences |
| Mouse IgG2a κ Isotype Control | A647 | G155-178 | BD Biosciences |

**Table 3. Structure of the two different 2^{nd} generation CARpTα constructs generated in this invention.**

| | **CARpTα.41BB** | **CARpTα.28** |
|---|---|---|
| Antigen-binding domain | anti-pTα scFv | anti-pTα scFv |
| Hinge domain | Human CD8α | Human IgG4 hinge (with extra spacer IgG1 CH3) |
| Transmembrane domain | Human CD8α | Human CD28 |
| Signalling domains | Human 4-1BB and CD3ζ | Human CD28 and CD3ζ |

## Claims

1. An isolated antibody, or an antigen binding fragment thereof, comprising a heavy chain (HC) immunoglobulin variable domain sequence and a light chain (LC) immunoglobulin variable domain sequence, wherein
the HC comprises one or more of
(a) a CDR1 comprising the amino acid sequence of RYAMS (SEQ ID NO: 1) or an equivalent of each thereof; and/or
(b) a CDR2 comprising the amino acid sequence of AAISSGGSYTYYPDSVKG (SEQ ID NO: 2) or an equivalent of each thereof; and/or
(c) a CDR3 comprising the amino acid sequence of LRVVAEGGSYFDY (SEQ ID NO: 3) or an equivalent of each thereof; and/or
the LC comprises one or more of:
(a) a CDR1 comprising the amino acid sequence of RSSQSILHSNGNTYLE (SEQ ID NO: 4) or an equivalent of each thereof; and/or
(b) a CDR2 comprising the amino acid sequence of KVSNRFS (SEQ ID NO: 5) or an equivalent of each thereof; and/or
(c) a CDR3 comprising the amino acid sequence of FQGSHVPVT (SEQ ID NO: 6) or an equivalent of each thereof.

2. The isolated antibody according to claim 1, wherein the HC immunoglobulin variable domain comprises the amino acid sequence SEQ ID NO: 7 and/or LC immunoglobulin variable domain comprises the sequence SEQ ID NO: 8.

3. The isolated antibody according to claim 1 or 2, wherein the antibody is selected from the group consisting of a monoclonal antibody, a chimeric antibody, and a humanized antibody, or wherein the antigen binding fragment is selected from a Fab, a F(ab')2, a Fab', a scFv, and a Fv.

4. The isolated antibody according to any one of claims 1 to 3, wherein the antibody wherein is coupled to a toxin.

5. A chimeric antigen receptor (CAR) comprising:
(a) an extracellular domain comprising a heavy chain (HC) immunoglobulin variable domain sequence and a light chain (LC) immunoglobulin variable domain sequence of any one of claims 1 to 4,
(b) a transmembrane domain; and
(c) at least one endodomain.

6. CAR according to claim 5, further comprising a hinge domain between domain (a) and domain (b).

7. The CAR according to claim 5 or 6, wherein
- the hinge domain is a CD8α hinge domain, and/or
- the transmembrane domain is a CD8α transmembrane domain; and/or
- the endodomain comprises a CD3ξ signaling domain alone or in combination with a CD28 coestimulatory domain or a 41BB coestimulatory domain.

8. An isolated nucleotide sequence encoding the antibody according to any one of claims 1 to 4, or the CAR according to any one of claims 5 to 7.

9. The isolated nucleotide sequence of claim 8, further comprising a Kozak consensus sequence, peptide signal sequence and/or an enhancer sequence, preferably, these sequences are located upstream of the sequence encoding the antigen binding domain of the antibody.

10. A vector comprising the isolated nucleotide sequence according to claim 8 or 9, preferably, the vector is a plasmid, a retroviral vector, a lentiviral vector, an adenoviral vector, and an adeno-associated viral vector.

11. A cell comprising
- the CAR according to any one of claims 5 to 7; and/or
- the isolated nucleotide sequence according to claims 8 or 9; and/or
- the vector according to claim 10,
wherein the cell is, preferably, a T-cell.

12. A composition comprising
- the antibody according to any one of claims 1 to 4; and/or
- the CAR according to any one of claims 5 to 7; and/or
- the isolated nucleotide sequence according to claims 8 or 9; and/or
- the vector of claim 10; and/or
- the cell according to claim 11.

13. A kit comprising
- the antibody according to any one of claims 1 to 4; and/or
- the CAR according to any one of claims 5 to 7; and/or
- the isolated nucleotide sequence according to claim 8 or 9; and/or
- the vector of claim 10; and/or
- the cell according to claim 11, and/or
- the composition according to claim 12.

14. The antibody according to any one of claims 1 to 4; the CAR according to any one of claims 5 to 7; the isolated nucleotide sequence according to claim 8 or 9; the vector according to claim 10; the cell according to claim 11; the cell according to claim 12; or the kit according to claim 13, for use in as a medicament, preferably, for use in the treatment and/or prevention of leukemia, mores preferably T-cell acute lymphoblastic leukemia (T-ALL), still more preferably T-ALL pre-TCR+, in a subject.

15. A method of producing anti-pTα CAR T-cells comprising:
(i) introducing a nucleotide sequence encoding the CAR according to claim 8 or 9 in a population of T-cells; and
(ii) selecting a subpopulation of T-cells that have been successfully transduced with said nucleotide sequence of step (i) thereby producing anti-pTα CAR T-cells.

16. An *in vitro* method for detecting a leukemia cell in a sample isolated from a subject, comprising
(a) analysing
- the levels of pTα in the biological sample by detecting the complex formed by the antibody according to any one of claims 1 to 4, or by the antigen binding fragment thereof, and pTα; or alternatively
- the proportion of cells in a biological sample showing on the surface the complex formed by the CAR according to any one of claims 5 to 7, or the antigen binding fragment thereof, and pTα, or the complex formed by the T-cells which express on its surface the CAR according to any one of claims 5 to 7 and pTα,
and
(b) comparing
- the levels of pTα observed in step (a) with the levels of pTα observed in a control biological sample; or
- the proportions of pre-TCR+ cells observed in step (a) with the proportions of pre-TCR+ cells observed in a control biological sample,
wherein the leukemia cell is detected when
- the level of pTα is elevated compared to that observed in the control biological sample, or
- the proportion of pre-TCR+ cells is elevated compared to that observed in the control biological sample;
and
wherein the sample isolated from a subject is not from thymus.
